# EUROPÄISCHE PATENTSCHRIFT

(11) **EP 2 471 955 B1**
(45) Veröffentlichungstag und Bekanntmachung des Hinweises auf die Patenterteilung: **27.07.2016**
(21) Anmeldenummer: 12153295.6
(22) Anmeldetag: 21.02.2008
(51) Int. Cl.: C12Q 1/68

(54) **Organismusspezifisches hybridisierbares Nucleinsäuremolekül**
Organism-specific hybridisable nucleic acid molecule
Molécule d'acide nucléique hybridisable spécifique à l'organisme

(30) Priorität: 23.02.2007 DE 102007010311
(43) Veröffentlichungstag der Anmeldung: 04.07.2012
(62) Teilanmeldung aus: 08715931.5
(73) Patentinhaber: Identxx GmbH, 70469 Stuttgart (DE)
(72) Erfinder: Thines, Marco, Prof. Dr., 70435 Stuttgart (DE); Brändle, Frank, 70191 Stuttgart (DE)
(74) Vertreter: Witte, Weller & Partner Patentanwälte mbB

(56) Entgegenhaltungen:
- EP-A- 0 751 227
- WO-A-01/11075
- WO-A-95/29260
- ERSEK T ET AL: "PCR AMPLIFICATION OF SPECIES-SPECIFIC DNA SEQUENCES CAN DISTINGUISH AMONG PHYTOPHTHORA SPECIES", APPLIED AND ENVIRONMENTAL MICROBIOLOGY, WASHINGTON,DC, Bd. 60, Nr. 7, 1. Juli 1994 (1994-07-01), Seiten 2616-2621, XP001087933, ISSN: 0099-2240
- MARTIN F. & KISTLER H.C.: "species specific banding patterns of restriction endonuclease-digested mitochondrial DNA from genus Pythium", EXP. MYCOLOGY, Bd. 14, 1990, Seiten 32-46, XP002480036,
- BOROVKOV A Y ET AL: "A tandemly repeated sequence from the Plasmopara halstedii genome", GENE, ELSEVIER, AMSTERDAM, NL, Bd. 124, Nr. 1, 14. Februar 1993 (1993-02-14), Seiten 127-130, XP023540689, ISSN: 0378-1119, DOI: 10.1016/0378-1119(93)90772-U [gefunden am 1993-02-14]
- ZHANG Z ET AL: "Molecular detection of Fusarium oxysporum f. sp. niveum and Mycosphaerella melonis in infected plant tissues and soil", FEMS MICROBIOLOGY LETTERS, NO LONGER PUBLISHED BY ELSEVIER, Bd. 249, Nr. 1, 1. August 2005 (2005-08-01), Seiten 39-47, XP027871967, ISSN: 0378-1097 [gefunden am 2005-08-01]
- P.H. GOODWIN: 'cloned dna probes for identification of phytophthora parasitica' PHYTOPATHOLOGY Bd. 79, Nr. 6, 01 Januar 1989, Seiten 716 - 721, XP055076982
- BOROVKOV A Y ET AL: "A tandemly repeated sequence from the Plasmopara halstedii genome", GENE, ELSEVIER, AMSTERDAM, NL, vol. 124, no. 1, 14 February 1993 (1993-02-14), pages 127-130, XP023540689, ISSN: 0378-1119, DOI: 10.1016/0378-1119(93)90772-U [retrieved on 1993-02-14]

### Sequenzprotokoll

<110> Thines, Dr. Marco
<120> Spezifisches hybridisierbares Nucleinsäuremolekül
<130> 4061P100WO
<160> 39
<170> PatentIn Version 3.3
<210> 1
   <211> 812
   <212> DNA
   <213> Peronospora arborescens
   <220> Keines
   <400> 1
   cggccgccag tgtgctggaa ttcgccctta ggtcgcgggt tcgaatccac gccctttcgg     60
   accagtgcct taaactggcg ccttagaccg ctcggccaat tgaccgtcaa ccacaaacca    120
   caaccctcaa ccgtacacaa aacatacaca aaccacaaca actcattaca actcaacgca    180
   cacctcgctc gccaaataca cagtcgacac gtgcaagaac tggccaaaca tgccgccacc    240
   cttctccaca actcgctcca ccatcgtttc tgtctgctta cgatgccgtc ggcaagaact    300
   gggtgactgc ctgtcgacag catagcgggc ctttatgctg ctgtagtaga agtgatgcac    360
   gattgtactg aaggcaccac gcactaagct atgaccacca caccaaatcg atcgatcttc    420
   gtggcgagcc agagtgacct acatctgtca gtccctgcat cagtccggga tgagtccagc    480
   ctcgcatctt ctggcgcatg ctgctcacga taacaagtaa accaactatt acctctactg    540
   acagcagcgg atatgcagct tatagtgtag cacagtcgag gagcagcgtc cgaagtgcag    600
   caacgtgaat ttgacaaagg tttgatatca aaagcaggtg tagtcgccgg acttgagccc    660
   acgcgaaagc aagtagtcag ttagctggtc cctccggtgt gacatagctc aatcaacagc    720
   atcaaagacg cgtgttcgcg agatataggg gaatggctgg agcataatag aaaagaatga    780
   gttgcccaac ctccttggga ttcgataccc gc                                  812
<210> 2
   <211> 784
   <212> DNA
   <213> Peronospora swinglei s.l.
   <220> Keines
   <400> 2
   gtcgtcgtcg tcgtccatct caatgctgga catggctgct ctcgcaggtc tgactaccat     60
   gaacgatgca gtatctccaa ctggatcaac ctactcgact tcctcttcca tgtcgtactc    120
   gtcactggct tcacgtgatg tcatgtacac acccgactcg gtcgactgca gtcttaacga    180
   gaccgcgtgg tctggtctac ccgttggcgg tgcttcgtac ttgatcgagt ttgacgacat    240
   gcccctatct atgagcagcc accagagctt cttatcttca gacgagatcg agtttctgtg    300
   ctctgccatc cttgagtaat tcgcaagcac aagaagcaaa acgcctccct cagcattcta 360
   agaggtcatg caaacaagct tttgttccgt cgaacccggt tggcaagtgc tgctgtcggc 420
   ttcaatctct cgagcgtata gtacgtagca cattaagttt agagcattat aattcttcat 480
   tgtagacttc gttgtataaa gtcataatgg aagggatatc tatctatcaa caaaaagctt 540
   gcaacctttc gtattgaacg ctatttgatt tcattggcga ctaatgctag cgaggtatca 600
   ttgctaccag tagagccact ggtttgtcta accgatagca gccgtcaatg aacgacagca 660
   cctgccgtca tgaggaccca ctcgttgaag tctccatggc atataaaaaa cgctaccatg 720
   accaatccaa caggaccagc ctcatcttga aattggcaga ggtagggatt tgcgtgcacc 780
   ggac 784
<210> 3
   <211> 619
   <212> DNA
   <213> Fusarium oxysporum
   <220> Keines
   <400> 3
   aagggaacaa aagctgggta ccgggccccc cctcgaggtg acggtatcga taagcttgat 60
   atccactgtg gaattcgccc ttgacagaca gacagacagc tcacggccaa cagcatacag 120
   gaaatttgtt tcttgcagcg ttgccatggc ctggtatccc ccccacacaa tccttctcaa 180
   taatagctgt ttaggtacac cctcctcctt cacgggactt tcaaacgggt tccccaatct 240
   caatcccgag accttctcga ctcagtacgc ctcggtctct ttctagatgt tatctgaaca 300
   agcttyaatt gctctttgag ctcctatgca tagttaggat cattgccttt gataaatcta 360
   tctctggggc accgacctcg atttcattga gccgctgggt ggccatgtcc gaactctttg 420
   acaaatcttt ataaatatca cctatgttaa cagtaccaag cgcaagactt gaaagactgt 480
   cgagggttgt gctgctgttt tcattcacat ctttccggtc cttgtctgtc tgtctgtcaa 540
   gggcgaattc cacattgggc tgcagcccgg gggatccact agttctagag cggccgcacc 600
   gcgggagctc caattcgcc 619
<210> 4
   <211> 782
   <212> DNA
   <213> Fusarium oxysporum
   <220> Keines
   <400> 4
   aagctgggta ccgggccccc cctcgaggtc gacggtatcg ataagcttga tatccactgt      60
   ggaattcgcc cttaggtcgc gggttcgaat ccccataggt atgtgttatc tgacnagggg     120
   gcctattctc ttatccctct tactgatttt gatggtgaag cttgcgatgc atcgcgtgga     180
   atccgctcgg gactctagta gcaacggggt cgtcagagaa aaccctcaga gtgtgtaagt     240
   tgatcaagtc tgaaggcctg tccggccact agagcgaagc tgaatgagat agggaaccct     300
   gaaaagcctc atgtgaagtt ttcaacggag ctcaagggcc actcggcatc tgttgagaag     360
   gtcgcattca acccaacaaa agatgcagag ctttgcagtg tgagcagtga tggcgtggtc     420
   aagttctggg acgtgaggac aaaagcatgc ttcaatgagg tcaaaggcct tggggacgct     480
   tttactcttg tctgggcacc agatggaaac acgcttctcg ttgggaacaa ggcagatacc     540
   atcttcgttc tgtngccatc tcagtcgacg cctctcagca cgcatcagca gtccgtgcag     600
   accaaccaga tcgcattctg ntggagcggt gagaagatct tcctcacgac agctgaagga     660
   agtattcgag tcttgtcgta ccctggatta gaacccgcga cctaagggcg aattccacat     720
   tgggctgcag cccgggggat ccactagttc tagagcggcc gcaccgcggg agctccaatt     780
   cg                                                                    782
<210> 5
   <211> 231
   <212> DNA
   <213> Plasmopara petroselini
   <220> Keines
   <400> 5
   accaagagcg cctaacttga cgcttgtctg aaatccgacc gtcaactcat aggcgaatgc      60
   aaatttagtt taccagtatt tgatcatcat acatggcgtg taagacgtct aataatgaag     120
   acgcaagatt gtggagcgta aggagtcctt ttctcgtccg tgcaagcgcc gttggtttag     180
   tggtaaaata tctcgttgcc aacgagatgc cccggattcg aacccgcgac c              231
<210> 6
   <211> 691
   <212> DNA
   <213> Plasmopara petroselini
   <220> Keines
   <400> 6
   atgtagaaga agacaggcag gcagactcat aagtaaatgc gcattatcgc tctaggaagc      60
   gacactactg tacattttcc cgsacctata tacatacatg cacatactac acagtgccag     120
   ctacgcacat ggaacgacct agattacctt acccttcttg ccaatctcgc ctccctagtc     180
   attggccagt gacaggtgac cagtggctcg gatctgcatc gccacgatgc ttggtatgcc     240
   acatgcataa tctgcgacac atggccgccc catggcttgg tgtatggctt ggtgttcccc     300
   catgcaagcc atagccgtca cgttgctgtg ctctggtcac aatcgagatg ttccaggggc     360
   aagtaaacac gcggctatgc ggctcgaaaa gctcgcctaa gcaaagcgcc acgcaaagtc     420
   tggaggagcg ctgaacggaa gaggcggcga cggcagtcca ctacacttcg agaaaatgaa     480
   ttttagcttt ggtgcccggc agatgaggcg cttccacact ttcgagaggc tccgcggcta     540
   cgtctagttc gttagggccc gtatacgagt cggaagggca gcgaggtatg attcttgcgt     600
   gcaccggtgc agtgctaaac gcggcacatc acattcgggc ctagcgacga aggcaatagt     660
   gcgaaaaagg ttgccgctcc cgtctcgccc g                                    691
<210> 7
   <211> 317
   <212> DNA
   <213> Peronospora valerianellae
   <220> Keines
   <400> 7
   gacagattgc gttacccact ggctaaaaag aatacaagac atacgaaaga catgacagtt      60
   gactcactct ctctatgtgt gtgtgtgatt tcgttggtgt agatttctcg tgtgccattg     120
   cgtcgacgtt ggcgatgcac ggacgcatgt atccaacgag ttcccacgtg tgtttctact     180
   cgaatgtgtt tggacgagag agaaaggtca ggaaggtagc gaggttgaac cttgacgaga     240
   cactcactta gagcgctctc ttgggatttg cagcagcaac acgttttgtc ggcttttggt     300
   gtaatgttga tctgttg                                                    317
<210> 8
   <211> 463
   <212> DNA
   <213> Peronospora valerianellae
   <220> Keines
   <400> 8
   gaatccgacg ttttgggagg tctagaggca gcgacgtaca aatcaccccg atgtcgcgtg      60
   aatgctgtcc atatatgctg gacactggct tgggctagtg ttgtacaaag atactccggc     120
   atgacagtgt cgtgagacga gttcggcaat gctcaccaga ttgttcgacg gaaaatacga     180
   cggagccgtg tgcttacgat ctcagacctt gatacccgta ctactactgt ctcgagactg     240
   taccatcgat ctaggtcgac cagaccaagc gaagctcatg tctgtgtaac ttgggcgaga     300
   gcattcccgc tggagcagca ttgttgaagc tcctcgccag gtagtagcac tcgctgttca     360
   tttcacgctg acagggttac cgcaaatccc tcagacggca cacaaaagcg cgcaacgaat     420
   gatagcgctt gtgaacgtca gcagaagcac ggcgtcgcaa cca                       463
<210> 9
   <211> 17
   <212> DNA
   <213> Künstliche Sequenz
   <220> Keines
   <400> 9
   acgccctttc ggaccag 17
<210> 10
   <211> 18
   <212> DNA
   <213> Künstliche Sequenz
   <220> Keines
   <400> 10
   cagcatgcgc cagaagat 18
<210> 11
   <211> 22
   <212> DNA
   <213> Künstliche Sequenz
   <220> Keines
   <400> 11
   tgctctcgca ggtctgacta cc 22
<210> 12
   <211> 22
   <212> DNA
   <213> Künstliche Sequenz
   <220> Keines
   <400> 12
   cacgcaaatc cctacctctg cc 22
<210> 13
   <211> 22
   <212> DNA
   <213> Künstliche Sequenz
   <220> Keines
   <400> 13
   gtcttaacga gaccgcgtgg tc 22
<210> 14
   <211> 22
   <212> DNA
   <213> Künstliche Sequenz
   <220> Keines
   <400> 14
   atgacggcag gtgctgtcgt tc 22
<210> 15
   <211> 18
   <212> DNA
   <213> Künstliche Sequenz
   <220> Keines
   <400> 15
   ctctgtccac gcattccg 18
<210> 16
   <211> 22
   <212> DNA
   <213> Künstliche Sequenz
   <220> Keines
   <400> 16
   gtttgtatct gcttaaactg ac 22
<210> 17
   <211> 18
   <212> DNA
   <213> Künstliche Sequenz
   <220> Keines
   <400> 17
   gggttcccaa tctcaatc 18
<210> 18
   <211> 18
   <212> DNA
   <213> Künstliche Sequenz
   <220> Keines
   <400> 18
   actcactacg cctcggtc 18
<210> 19
   <211> 20
   <212> DNA
   <213> Künstliche Sequenz
   <220> Keines
   <400> 19
   tcgacagtct ttaagtcttg 20
<210> 20
   <211> 24
   <212> DNA
   <213> Künstliche Sequenz
   <220> Keines
   <400> 20
   accaagagcg cctaacttga cgct 24
<210> 21
   <211> 24
   <212> DNA
   <213> Künstliche Sequenz
   <220> Keines
   <400> 21
   gggttcgaat ccggggcatc tcgt 24
<210> 22
   <211> 23
   <212> DNA
   <213> Künstliche Sequenz
   <220> Keines
   <400> 22
   ccgtcaactc ataggcgaat gca 23
<210> 23
   <211> 24
   <212> DNA
   <213> Künstliche Sequenz
   <220> Keines
   <400> 23
   cactaaacca acggcgcttg cacg 24
<210> 24
   <211> 24
   <212> DNA
   <213> Künstliche Sequenz
   <220> Keines
   <400> 24
   cgcattatcg ctctaggaag cgac 24
<210> 25
   <211> 24
   <212> DNA
   <213> Künstliche Sequenz
   <220> Keines
   <400> 25
   ggcccgaatg tgatgtgccg cgtt 24
<210> 26
   <211> 24
   <212> DNA
   <213> Künstliche Sequenz
   <220> Keines
   <400> 26
   ccagctacgc acatggaacg acct 24
<210> 27
   <211> 23
   <212> DNA
   <213> Künstliche Sequenz
   <220> Keines
   <400> 27
   acgcaagaat catacctcgc tgc 23
<210> 28
   <211> 24
   <212> DNA
   <213> Künstliche Sequenz
   <220> Keines
   <400> 28
   gacagattgc gttacccact ggct 24
<210> 29
   <211> 24
   <212> DNA
   <213> Künstliche Sequenz
   <220> Keines
   <400> 29
   ccgacaaaac gtgttgctgc tgca 24
<210> 30
   <211> 24
   <212> DNA
<213> Künstliche Sequenz
   <220> Keines
   <400> 30
   ttgcgtcgac gttggcgatg cacg 24
<210> 31
   <211> 24
   <212> DNA
   <213> Künstliche Sequenz
   <220> Keines
   <400> 31
   gagcgctcta agtgagtgtc tcgt 24
<210> 32
   <211> 24
   <212> DNA
   <213> Künstliche Sequenz
   <220> Keines
   <400> 32
   gacgtacaaa tcaccccgat gtcg 24
<210> 33
   <211> 21
   <212> DNA
   <213> Künstliche Sequenz
   <220> Keines
   <400> 33
   tgctgacgtt cacaagcgct a 21
<210> 34
   <211> 24
   <212> DNA
   <213> Künstliche Sequenz
   <220> Keines
   <400> 34
   gatactccgg catgacagtg tcgt 24
<210> 35
   <211> 24
   <212> DNA
   <213> Künstliche Sequenz
   <220> Keines
   <400> 35
   gggatttgcg gtaaccctgt cagc 24
<210> 36
   <211> 18
   <212> DNA
   <213> Künstliche Sequenz
   <220> Keines
   <400> 36
   aggtcgcggt tcgaatcc 18
<210> 37
   <211> 749
   <212> DNA
   <213> Künstliche Sequenz
   <220> Keines
   <400> 37
   aattccaata tctttgtgaa ccgtttactt agtttttcgt ttgttttttt aataatataa 60
   tatcgttcgt ttccaaacaa tttttcaata ttttttggat taatatatta tataattcga 120
   aatatttttg gttccaataa acgaaattct tttggtattt ataaaaaaag aatttcccgt 180
   tttctttata ttggttaata attttaaata tttatatttt tcgttaaatt attattatta 240
   tttccggatt tttaaaagac ccgtaaaaat gtttcgatat ttttttaaaa aacgccggta 300
   attttaatat tcgtcgtcgt tttggaaatc caatttctgt cacagacctg aaggacagcc 360
   actgggctgt cgcttagtca tgacccctgt cacgtgaagg cgcgagggcc gagcgcctcg 420
   cgcgcgtata tctacgcgaa atctctgcag tctccgatat gtagctcctc gagctacgtc 480
   ttcgtcgaca accacctgct acactgtacc tggaagacta gatagccaat atactctgtc 540
   ctgttacctg atcgcccgca cctacctgtc cgccctgcct gcccgtgaca ttacgtagct 600
   ccttcattag gtgcccgcga tgcctgcgtc actgcaaccc ccgatcttaa ccgattcgac 660
   cgaggaactg atcgaacacc tacaaggaca ccctgaacaa tgggtgtctt acatctccga 720
   ttcctaccaa aagctgcaac tattgcacg 749
<210> 38
   <211> 751
   <212> DNA
   <213> Künstliche Sequenz
   <220> Keines
   <400> 38
   ggatccactc tgtccacgca ttccggcagc tttgagtttt ggttagtggt ttccagcatc 60
   gatgggtacc accatcgcat ttgaatcgat attgctgtca tttgagccta tgcgagcact 120
   tgtatcgcaa tgtcagacac caatgtccat acagcaacgt cgcttcaaat ctatgcgacc 180
   acttgtttgc gtttcattgt gaagcatcgt tagacaaatc aactttgtcc atgcattctt 240
   gcagcttggg aaatgcttag ttgttatcaa tctccatgtt tagcatctcc aggacaaaca 300
   caacgtgtag cattaccagg ccaaagtaaa tgttcataag caatagccgt tgctaacagc 360
   ttaaataaat gcaaacactt gttggcagtt cactgtttag cgtctttaag caaaaccact 420
   ttgttcatgc atcctcaaag ccgtgaacat gctatgtggt tggcagtgtc caacttttgc 480
   atctccaagc caatcacaat aatcattagc aatcgccatt gctatcagcc cgattagatg 540
   caaacacttc gcagtcactg taaagcgtct ttgagcgaat ttacattgtc catgcatcct 600
   cacagtcatg aacagtttaa gtggttgcca gcgttcatgt gtagaagcac tagaccaaat 660
   ataatgttca tcagcaatcg ccaatgctgt cagtttaagc agatacaaac acttgtttgg 720
   tgtcgactgt taagcgactt tgaacggatc c 751
<210> 39
   <211> 15
   <212> DNA
   <213> Künstliche Sequenz
   <220> Keines
   <400> 39
   gtcgtcgtcg tcgtc 15

## Beschreibung

Die vorliegende Erfindung betrifft ein Verfahren zur Auswahl eines organismusspezifischen hybridisierbaren Nucleinsäuremoleküls, ein nach diesem Verfahren hergestelltes Nucleinsäuremolekül, und die Verwendung einer intergenischen Nucleotidsequenz, die zwischen zwei Elementen auf der genomischen Information eines Organismus liegt, zur Herstellung eines organismusspezifischen hybridisierbaren Nucleinsäuremoleküls.

Unter Hybridisierung wird die Zusammenlagerung zweier Nucleinsäure-Einzelstränge, die komplementäre Basensequenzen besitzen, zu Doppelsträngen verstanden. Hybridisierung kann zwischen zwei DNA-Strängen, einem DNA- und einem RNA-Strang sowie zwischen zwei RNA-Strängen erfolgen.

Hybridisierbare Nucleinsäuremoleküle sind bspw. Primer für die Polymerase-Kettenreaktion (PCR-Primer) oder für andere Amplifizierungsverfahren, die auf der Aktivität einer Nucleinsäurepolymerase basieren, sowie Hybridisierungssonden. Bei den Primern, bspw. für die PCR, oder Hybridisierungssonden kann es sich um DNA-, RNA-oder synthetische Oligonucleotidmoleküle handeln, die zum Nachweis, zur Charakterisierung oder Lokalisierung von solchen Zielnucleinsäuremolekülen verwendet werden können, die eine Nucleotidsequenz aufweisen, welche zu jener der PCR-Primer oder Hybridisierungssonden komplementär ist. Verfahren zur Herstellung von PCR-Primern oder Hybridisierungssonden sind im Stand der Technik allgemein bekannt; vgl. Sambrook und Russel (2001), Molecular Cloning - A laboratory Handbook, 3. Auflage, Cold Spring Harbor Laboratory Press, New York. Der Nachweis der Zielnucleinsäuremoleküle ermöglicht einen indirekten Nachweis des Organismus, der das Zielnucleinsäuremolekül trägt. Auf diese Art lassen sich bspw. pathogene Organismen nachweisen, die das Zielnucleinsäuremolekül als Bestandteil ihrer genomischen Information enthalten. Die Identifizierung von pathogenen Organismen wiederum ist die Voraussetzung für eine zielgerichtete Therapie einer durch den Organismus ausgelösten Krankheit.

Die moderne Phytopathologie befasst sich mit der Erkrankung von Pflanzen durch abiotische Ursachen oder biotische Krankheitserreger. Zu diesen Krankheitserregern zählen Pflanzenviren, Bakterien, Pilze oder parasitische Blütenpflanzen. Gegenstand der phytopathologischen Forschung ist bspw. die Entwicklung von Nachweisverfahren, mit denen Krankheitserreger im Saat- und Pflanzengut, in Bodenproben, Flüssigsubstraten und beliebigen anderen Medien detektiert werden können. Herkömmliche Testverfahren der Phytopathologie beschränken sich im Wesentlichen auf das Auspflanzen von möglicherweise erkrankten Pflanzen und die visuelle Charakterisierung des Phänotyps hinsichtlich charakteristischer Krankheitsmerkmale (Bonitur). Diese auf visueller Bonitur basierenden Nachweisverfahren für Pflanzenerkrankungen sind jedoch sehr zeit- und arbeitsaufwändig.

Alternativ kommen serologische Verfahren zum Nachweis von Phytopathogenen zum Einsatz, bei denen gegen die Pathogene gerichtete Antikörper verwendet werden, bspw. im Rahmen eines sogenannten "enzyme linked immunosorbent assay" (ELISA). Serologische Verfahren sind jedoch sehr kostenintensiv, da die Herstellung von spezifischen monoklonalen Antikörpern mit hohem technischem Arbeitsaufwand verbunden ist. Auch ist es wegen der großen Homologien der antigenen Strukturen von eukaryotischen Pathogenen häufig sehr schwierig, hochspezifische Antikörper gegen Eukaryoten zu entwickeln, die keine Kreuzreaktion mit nahe verwandten Spezies oder *mit formae specialis*, d.h. hochspezialisierten Unterarten einer Spezies, aufweisen.

Bei Nachweisverfahren, die sich der Polymerase-Kettenreaktion (PCR) bedienen, werden unter Verwendung von entsprechenden PCR-Primern üblicherweise moderat variable Genomabschnitte des Pathogens amplifiziert, wie der sog. "internal transcribed spacer" (ITS). Diese moderat variablen Genomabschnitte der Pathogene weisen über die Artgrenzen hinweg sehr große Sequenzhomologien auf. Daraus resultiert, dass sich mit dieser Methode sehr nahe verwandte Arten von Pathogenen kaum unterscheiden lassen. Ferner ist mittels dieser herkömmlichen PCR-gestützten Verfahren eine Differenzierung von solchen Schaderregern innerhalb derselben Art, die verschiedene Wirte befallen oder besiedeln bzw. *von formae specialis*, nicht möglich.

Aus der WO 95/33075 und der DE 698 27 934 sind Verfahren zur Identifizierung des Ursprunges von DNA-Proben aus DNA-enthaltenden Organismen beschrieben. Dazu werden von den zu analysierenden DNA-Proben aus DNA-Amplifikaten bestehende genetische Fingerabdrücke erstellt und mit genetischen Fingerabdrücken von DNA-Proben aus Referenzorganismen verglichen. Dieser sogenannte "Mustervergleich" ist jedoch nachteilig. Die zu vergleichenden Muster sind meist nicht genau identisch, so dass ein Vergleich äußerst schwierig ist. Der Aufwand ist groß und für eine schnelle Identifikation, wie sie die landwirtschaftliche Praxis fordert, nicht geeignet.

Borovkov und McClean, Gene, 124 (1993), 127-130, schlagen eine 747-bp lange Nucleotidsequenz aus dem Genom des pflanzenpathogenen Pilzes *Plasmopara halstedii* als Sonde zur Unterscheidung von Rassen dieses Pathogens vor.

Vor diesem Hintergrund ist es eine Aufgabe der vorliegenden Erfindung, ein Verfahren bereitzustellen, mit dem die Auswahl von hochselektiven, organismenspezifischen, hybridisierbaren Nucleinsäuremolekülen gelingt, die im Rahmen eines Verfahrens zum Nachweis von biotischen Krankheitserregern eingesetzt werden können, und mit denen die zuvor genannten Nachteile aus dem Stand der Technik vermieden werden. Insbesondere sollen hochselektive, organismenspezifische Nucleinsäuremoleküle hergestellt werden, die eine reduzierte Kreuzreaktivität mit verwandten pathogenen Spezies oder *formae specialis* aufweisen.

Diese Aufgabe wird durch ein Verfahren mit den Merkmalen des Patentanspruchs 1 gelöst.

Unter einem "Organismus" wird ein jegliches Lebewesen verstanden. Erfindungsgemäss sind das Lebewesen, die einen pflanzlichen; Wirt besiedeln bzw. befallen können. Ggf. können dadurch bei dem Wirt Krankheiten ausgelöst werden. In diesem Zusammenhang wird an Pflanzenkrankheiten gedacht, die durch Pflanzenviren, Bakterien, Pilze, Oomyceten, parasitische Pflanzen und Tiere etc. ausgelöst werden.

Unter "genomischer Information" wird Nucleinsäure verstanden, die spezifisch für den Organismus ist. Erfindungsgemäss wird an das Genom in Form der DNA gedacht Erfindungsgemäß umfasst die genomische Information nicht nur informationstragende sondern auch inter- und/oder exogenische Abschnitte des Genoms.

Verfahren zur Isolierung der genomischen Information aus einem Organismus sind im Stand der Technik allgemein bekannt; vgl. Sambrook und Russell (a.a.O.).

Zur Erstellung des genetischen Fingerabdrucks werden in Schritt 2 des erfindungsgemäßen Verfahrens folgende Schritte durchgeführt: 2.1 Amplifizierung von Abschnitten der genomischen Information des Organismus zum Erhalt einer Mischung von Amplifikaten, und 2.2 Vereinzelung der in der Mischung enthaltenen Amplifikate zum Erhalt eines Musters von Amplifikaten von Genomabschnitten.

Unter einem "genetischen Fingerabdruck" werden charakteristische genetische Merkmale eines Organismus verstanden, welche dessen Identifizierung erlauben. Diese Identifizierungsmöglichkeit basiert auf dem Vorkommen bestimmter Nucleotidabfolgen in der genomischen Information des Organismus. Hierunter fallen bspw. repetitive, tandemartig angeordnete DNA-Sequenzen. Ein prominentes Beispiel für diese repetitiven DNA-Sequenzen ist die sogenannte Satelliten-DNA. Satelliten-DNA besteht aus einer Vielzahl von Kopien von Nucleotidabfolgen, die direkt hintereinander vorkommen. Beispiele von kurzen solchen Wiederholungsfolgen sind A, AC, AAT oder AAC, wobei auch andere Abfolgen beobachtet werden. Satelliten-DNA mit AC-Dinucleotid-Folgen kommt durchschnittlich einmal pro 30 kb-Genomabschnitt vor. Tri- oder Tetranucleotid-Folgen sind seltener, einmal unter einigen hunderttausend Basenpaaren. In der genomischen Information des Organismus werden ferner längere sich wiederholende Abschnitte von 5 bis 2000 Nucleotiden Länge gefunden. Diese repetitiven DNA-Sequenzen sind im Genom weitgehend statistisch verteilt, wobei sie an den Centromeren und Telomeren der Chromosomen gehäuft auftreten.

Die Zahl der Di-, Tri- oder Tetranucleotide etc. in einer Satelliten-DNA ist hochpolymorph. D.h. zwei Allele unterscheiden sich mit hoher Wahrscheinlichkeit in der Zahl der Di-, Tri- oder Tetranucleotide einer gegebenen Satelliten-DNA. Die Ursache für diesen Polymorphismus sind Verrutschungen in DNA-Sequenzwiederholungen im Bereich der Replikationsgabeln. Die Satelliten-DNA wird nach den Mendelschen Regeln vererbt.

Verfahren zur Generierung von genetischen Fingerabdrücken, die im erfindungsgemäßen Schritt 2 zur Anwendung kommen, sind im Stand der Technik umfassend beschrieben und werden als "DNA-Fingerprinting", "DNA-Profiling" oder "DNA-Typing" bezeichnet; diese sind bspw. beschrieben in Weising et al. (1995), "Fingerprinting in Plants and Fungi", CRS Press, Boca Raton, Florida, USA; der Inhalt dieser Publikation ist durch Inbezugnahme Bestandteil der vorliegenden Anmeldung.

So ist eine große Zahl der repetitiven Abschnitte, wie bspw. Satelliten-DNA, mittlerweile sequenziert und Methoden zum Identifizieren weiterer Satelliten-DNA sind im Stand der Technik umfassend beschrieben; vgl. bspw. Kolpakov et al. (2003), "mreps: efficient and flexible detection of tandem repeats in DNA. Nucleic Acids Research 31 (13), Seiten 3672-3678. Der Inhalt dieser Publikation ist durch Inbezugnahme Bestandteil der vorliegenden Anmeldung. Das Computerprogramm "mreps", mit dem die Identifizierung von Satelliten-DNA gelingt, ist bspw. auf der Website "Bioinfo" der Bioinformatikgruppe an der "Universite des Sciences et Technologies de Lille", Frankreich unter http://bioinfo.lifl.fr/mreps/ oder auf der Website der "Bioinformation Systems e.V., Verein zur Förderung der Datenverwertung in der molekularen Biologie" unter http://www.bioinfo.de/isb/gcb01/poster/wu.html erhältlich. Weitere Computerprogramme zur Identifikation von Satelliten-DNA sind "SciRoKo", erhältlich auf der Website "www.kofler.or.at/Bioinformatics"; "MISA", erhältlich auf der Website "http://pgrc.ipk-gatersleben.de/misa/";"SSRFinder", erhältlich auf der Website http://www.maizemap.org/bioinformatics/SSRFINDER/SSR_Finder_Download.html; "SSRIT", erhältlich auf der Website "http://www.gramene.org/db/searches/ssrtool"; "TRF", erhältlich auf der Website "http://cagt.bu.edu/page/TRF_download" ; "Sputnik", erhältlich auf der Website "http://espressosoftware.com/pages/sputnik.jsp".

Die Nucleotidsequenzen zahlreicher Satelliten-DNA sind öffentlich zugänglichen Datenbanken zu entnehmen. Satelliten-DNA aus Pflanzen, Tieren und Pilzen sind bspw. auf der Website des "International Crops Research Institute for the Semi-Arid Tropics (ICRISAT)" unter http://intranet.icrisat.org/gtl/ssr/AnnotatedSSRs.asp?dname =Sorghum veröffentlicht. Satelliten-DNA aus Pflanzen sind ferner auf der Website "Plant-Sat" des Labors für molekulare Cytogenetik, Institut für molekulare Pflanzenbiologie, Ceske Budejovice, Tschechische Republik, unter http://w3lamc.umbr.cas.cz/ PlantSat/ veröffentlicht.

Basierend auf diesen Sequenzinformationen lassen sich PCR-Primer herstellen, die an die Satelliten-DNA hybridisieren. Es versteht sich, dass der Vorwärts- und Rückwärtsprimer jeweils an die gleiche Satelliten-DNA oder aber auch an unterschiedliche Satelliten-DNA, d.h. unterschiedliche Nucleotidsequenzen, hybridisieren kann. Wichtig ist lediglich, dass die zwischen zwei solchen repetitiven Abschnitten der genomischen Information liegenden Abschnitte amplifiziert werden, die im Folgenden "Amplifikate" genannt werden. Dies erfolgt in dem gegebenen Beispiel mittels der PCR-Reaktion. Es versteht sich, dass weitere Methoden der Nucleinsäureabschnittsvervielfältigung in Frage kommen, wie bspw. Amplifizierung mittels phi-DNA-Polymerasen oder der thermophilic Helicase-Dependent Amplification (tHDA).

Die jeweiligen Amplifikate können dann erfindungsgemäß mittels chromatographischer oder elektrophoretischer Verfahren bspw. ihrer Größe nach aufgetrennt und über spezifische an DNA bindende oder assoziierende Farbstoffe sichtbar gemacht werden. Dadurch wird ein Muster von Amplifikaten erhalten, das aufgrund des angesprochenen Polymorphismus charakteristisch für den jeweils analysierten Organismus ist. Dieses Muster wird als genetischer Fingerabdruck bezeichnet. In einem Extremfall, wenn der oder die für das DNA-Fingerprinting genutzten Primer nur ein einzelnes Fragment der genomischen Information amplifizieren, kann erfindungsgemäß das Muster aus nur einem Amplifikat bestehen. Es versteht sich, dass mit "einem" Amplifikat erfindungsgemäß gemeint ist, dass mehrere Nucleinsäuremoleküle mit identischer Nucleotidsequenz vorliegen, die sich folglich voneinander nicht unterscheiden.

In dem nächsten erfindungsgemäßen Schritt 3 wird aus dem erhaltenen genetischen Fingerabdruck bzw. dem Muster von Amplifikaten "ein" Amplifikat isoliert. Dies kann bspw. dadurch erfolgen, dass eine Bande, die die Nucleinsäure "eines" Amplifikates enthält, aus einem Elektrophoresegel mit einem Messer herausgeschnitten wird, und die darin enthaltende Nucleinsäure nach allgemein bekannten Methoden aus dem Gel eluiert wird.

Die im nächsten erfindungsgemäßen Schritt 4 erfolgende Sequenzierung des isolierten Amplifikates wird ebenfalls gemäß im Stand der Technik allgemein bekannter Verfahren durchgeführt, bspw. mittels dem Maxam-Gilbert- oder dem Sanger-Verfahren; vgl. Sambrook und Russell (a.a.O.).

Nachdem die Nucleotidsequenz des isolierten Amplifikates erhalten wurde, wird im Schritt 5 ein solcher Abschnitt ausgewählt, der zur Herstellung eines hybridisierbaren Nucleinsäuremoleküls geeignet ist. Dieser Abschnitt weist 3 bis 1000 Nucleotide auf. Die hierbei verwendeten Auswahlkriterien sind dem Fachmann allgemein bekannt. Ist bspw. die Herstellung eines PCR-Primers bezweckt, so werden vorzugsweise Abschnitte ausgewählt, die eine Länge von 3 oder 10 bis 36 Nucleotiden, vorzugsweise 18 bis 25 Nucleotiden, aufweisen, allerdings auch kürzer oder länger sein können. Die vom Fachmann angelegten Kriterien zur Herstellung eines PCR-Primers sind bspw. zu finden in Sambrook und Russel (a.a.O.), Chapter 8, Table 8.3. Zusammenfassend lassen sich die Auswahlkriterien wie folgt darstellen:
- Der G+C-Gehalt sollte zwischen 40 % und 60 %, vorzugsweise zwischen 45 % und 55 %, liegen mit einer gleichmäßigen Verteilung von allen vier Nucleotiden entlang der Länge des Primers.
- Zur Erhöhung der Amplifizierungsfähigkeit ist es von Vorteil, wenn der Primer am 3'-Ende das Nucleotid G oder C aufweist. Optimal ist es, wenn die letzten beiden Nucleotide am 3'-Ende die Variationen GG; GC; CG oder CC aufweisen.
- Der PCR-Primer sollte nicht mit sich selbst hybridisieren können. Optimal ist es, wenn ≤ 3 gleiche Nucleotide hintereinander folgen.
- Bei der Auswahl von Vorwärts- und Rückwärtsprimern sollten diese nicht gegenseitig hybridisierungsfähig sein. Optimal ist es, wenn beide Primer ≤ 3 komplementäre Nucleotide aufweisen. Der Abstand der beiden Primer sollte so gewählt sein, dass das Amplifikat in dem jeweilig verwendeten Detektionssystem gut nachzuweisen ist. Optimum für Polyacrylamidgele: 100 - 1500 Nucleotide; Optimum für Agarosegele: 250 - 750 Nucleotide; Optimum für Real-Time PCR-Systeme: 100 - 250 Nucleotide.
- Um einen spezifischen Nachweis zu ermöglichen, sollte der Primer so aufgebaut sein, dass eine Hybridisierungstemperatur von ≥ 50°C möglich ist. Die für einen Primer geltenden Hybridisierungstemperaturen lassen sich mit dafür vorgesehener Software berechnen.

Die eigentliche Herstellung des speziespezifischen hybridisierbaren Nucleinsäuremoleküls erfolgt unter Anwendung von dem Fachmann bekannten Standardmethoden der Nucleinsäuresynthese; vgl. ebenfalls Sambrook und Russell (a.a.O.).

Die der Erfindung zugrunde liegende Aufgabe wird hiermit vollkommen gelöst.

Die Erfinder haben erkannt, dass Amplifikate, die bei der Erstellung eines genetischen Fingerabdruckes des Organismus erhalten werden, geeignete Matrizen für die Herstellung von hochselektiven organismusspezifischen und hybridisierbaren Nucleinsäuremolekülen, wie bspw. Sonden und PCR-Primer, sind. So haben die Erfinder festgestellt, dass im Rahmen des DNA-Fingerprintings Fragmente der genomischen Information des interessierenden Organismus amplifiziert werden können, die in der Regel zwischen den Genen des Organismus, d.h. intergenisch, angeordnet sind, d.h. keine proteincodierende Information tragen. Diese Fragmente weisen aufgrund des geringen oder gar fehlenden Selektionsdrucks eine sehr hohe Variabilität zwischen einzelnen Organismen und somit eine hohe Spezifität für den jeweiligen Organismus auf. Mittels des erfindungsgemäßen Verfahrens lassen sich solche Nucleinsäuremoleküle gewinnen, die hoch-selektiv an derartige Fragmente binden können. Beispiele solcher Nucleinsäuremoleküle sind Sonden und PCR-Primer bzw. DNA-Oligomere. Unter Anwendung von im Stand der Technik bekannter Verfahren kann ein Fachmann, wie ein Molekularbiologe oder Pflanzenpathologe, feststellen, ob ein solches erfindungsgemäß hergestelltes Nucleinsäuremolekül an genomische Information in einer beliebigen biologischen Probe hybridisieren kann. Im Falle einer Hybridisierung kann darauf rückgeschlossen werden, dass der gesuchte Organismus in der Probe enthalten ist.

Besonders vorteilhaft ist, dass mit den PCR-Primern bzw. Hybridisierungssonden, die über das erfindungsgemäße Verfahren hergestellt wurden, nicht nur zwischen verschiedenen Spezies sondern sogar hochselektiv zwischen Individuen unterschieden werden kann. Dadurch wird auch der Nachweis von *formae specialis* bzw. von pathogenen Subspezies innerhalb einer Art ermöglicht und die Voraussetzungen für eine zielgerichtete Behandlung einer befallenen Pflanze geschaffen.

Bei dem Verfahren ist die genomische Information des Organismus doppelsträngige DNA.

Diese Maßnahme hat den Vorteil, dass sich die genomische, doppelsträngige DNA des Organismus zur Erstellung eines genetischen Fingerabdrucks besonders eignet. So sind im Stand der Technik eine Vielzahl von Methoden beschrieben, die die Erstellung des genetischen Fingerabdrucks aus der Basis von doppelsträngiger DNA ermöglichen; vgl. Weising et al. (a.a.O.). Diese beinhalten die eingangs näher dargestellte PCR-basierende Methode des DNA-Fingerprintings.

Die Amplifizierung in Schritt (2.1) erfolgt mittels einer Polymerase-Kettenreaktion (PCR), wobei als Primer-Ansatzstellen Abschnitte von Satelliten-DNA, insbesondere von Mikrosatelliten-DNA und simple sequence repeats (SSRs), geeignet sind.

Unter Satelliten-DNA werden hauptsächlich im Genom von Eukaryoten vorkommende, vielfach wiederholte, d.h. repetitive, DNA-Sequenzen verstanden, die je nach Organismus bis zu 90 % des Gesamtgenoms ausmachen können. Satelliten-DNAs werden anhand ihrer repetitiven Nucleotidsequenzlänge und der Anzahl dieser tandemartig angeordneten Kopien verschieden bezeichnet. Kleinere Satelliten-DNA wird oft als "Mikrosatelliten-DNA" bezeichnet. Darunter versteht man Di-, Tri- oder Tetranucleotide, die zu 10 bis 30 Kopien innerhalb einer "geclusterten" Repetitionseinheit auftreten. Mikrosatelliten-DNA umfasst auch repetitive Sequenzen von 20 bis 40 Nucleotiden und erstreckt sich über einen Bereich von einigen Kilobasen. Man nennt sie auch "variable number of tandem repeats" (VNTR). "Single sequence repeats" (SSRs), die auch als "simple sequence repeats" bezeichnet werden, sind kurze, repetitive, 1 bis 4 Nucleotide umfassende Sequenzen.

Diese Maßnahme hat den Vorteil, dass die Nucleotidsequenzen einer Vielzahl von Satelliten-DNAs im Stand der Technik beschrieben sind. Es ist somit für den Fachmann ein Leichtes geeignete PCR-Primer zu generieren, die an die Satelliten-DNA hybridisieren. Nucleotidsequenzen von Satelliten-DNAs finden sich bspw. auf der Website des "International Crops Research Institute for the Semi-Arid Tropics (ICRISAT)" (a.a.O.). Der Inhalt dieser Website ist durch Inbezugnahme Bestandteil der vorliegenden Anmeldung. Satelliten-DNA lässt sich allgemein mit (Nx)n darstellen, wobei N für ein beliebiges Nucleotid steht, und x eine ganze Zahl von 2-6 für SSRs, x eine ganze Zahl von 7-30 für Mikrosatelliten-DNA und x eine ganze Zahl von 31-10000 für Makrosatelliten-DNA ist; n ist allgemein eine ganze Zahl von 2 bis 1000, wobei sowohl für n als auch für x größere Werte in Betracht kommen.

Bei einem alternativen Verfahren kann im Rahmen der PCR als Vorwärts-Primer ein solcher gewählt werden, der an eine randomisierte Nucleotidsequenz mit einer Länge von 3 bis 30, vorzugsweise von 4 bis 20, höchst bevorzugt von 6 bis 10 Nucleotiden, auf dem ersten DNA-Strang des Organismus hybridisiert, und als Rückwärts-Primer ein solcher gewählt werden, der an eine Nucleotidsequenz mit einer Länge von 3 bis 30, vorzugsweise von 4 bis 20, höchst bevorzugt von 6 bis 10 Nucleotiden, auf dem zu dem ersten DNA-Strang komplementären DNA-Strang des Organismus hybridisiert.

Auch über diese Maßnahme lässt sich ein genetischer Fingerabdruck in Form eines Musters von Amplifikaten von Fragmenten der genomischen Information des interessierenden Organismus erstellen. Dazu wird ein Vorwärts-PCR-Primer hergestellt, der 3 bis 10 willkürlich ausgewählte Nucleotide aufweist. Der Rückwärts-PCR-Primer wird entsprechend gewählt. Die Erfinder haben herausgefunden, dass sich aus statistischen Gründen bei einer Vielzahl von Organismen entsprechende Nucleotidsequenzen über das Genom verteilt finden lassen. Im Rahmen der PCR-Reaktion werden dann die dazwischenliegenden Fragmente der genomischen Information des Organismus amplifiziert, die nach Erkenntnissen der Erfinder aus statistischen Gründen ebenfalls eine sehr hohe Organismusspezifität aufweisen, da ein Großteil des Genoms informationsfrei ist, und sich deshalb zur Herstellung von organismusspezifischen hybridisierbaren Nucleinsäuremolekülen eignen.

Bei einem weiteren alternativen Verfahren wird im Rahmen der PCR als Vorwärts-Primer ein solcher gewählt, der an eine auf ein Virusgenom zurückführbare Nucleotidsequenz auf dem ersten DNA-Strang des Organismus hybridisiert, und als Rückwärts-Primer wird ein solcher gewählt, der an eine auf ein Virusgenom zurückführbare Nucleotidsequenz auf dem zu dem ersten DNA-Strang komplementären DNA-Strang des Organismus hybridisiert.

Auch mit dieser Maßnahme lassen sich im Rahmen eines DNA-Fingerprintings Amplifikate von solchen Genomfragmenten erhalten, die die Herstellung speziespezifischer hybridisierbarer Nucleinsäuremoleküle ermöglichen. Auf ein Virusgenom zurückführbare Nucleotidsequenzen lassen sich aufgrund von Sequenzhomologien identifizieren. So wird von einer auf ein Virusgenom zurückführbaren Nucleotidsequenz gesprochen, wenn Abschnitte der genomischen Information von zumindest 10 Nucleotiden eine Homologie mit einem entsprechenden Abschnitt eines Virusgenoms von zumindest 30 %, vorzugsweise von zumindest 40 %, 50 %, 60 %, 70 %, 80 %, 90 %, 95 % und höchst bevorzugt von zumindest 100% aufweisen. Die Homologien lassen sich bspw. mittels der Software MegAlign des Lasergene Programms von DNAStar Inc. bestimmen.

Auf ein Virusgenom zurückführbaren Nucleotidsequenzen sind im Stand der Technik umfassend beschrieben, vgl. Cooper und Geoffrey (2000), "The Cell - A molecular approach", 2. Auflage, Sunderland (MA): Sinauer Associates, Inc.; Brown, T. A. (2002), "Genoms" 2. Auflage, New York und London: Garland Science; Pearson und Morrow (1981), "Discrete-length repeated sequences in eukaryotic genomes", Proceedings ofthe National Academy of Sciences of the United States of America, Vol. 78, Seiten 4016-4020; Bernardi, G. (2005), "Structural and evolutionary genomics - natural selection in genome evolution", Elsevier Science Publications, und Baltimore, D. (1985), "Retroviruses and retrotransposons: the role of reverse transcription in shaping the eukaryotic genome", Cell, 40 (3), Seiten 481-482. In dem dieser Anmeldung beigefügten Sequenzprotokoll wird unter SEQ ID-Nr. 37 ein Beispiel einer auf ein Virusgenom zurückführbaren Sequenz angegeben, aus der sich geeignete PCR-Primer ableiten lassen. Diese Sequenz stammt aus der Publikation von Shull und Hamer (1996), "Genetic differentiation in the rice blast fungus revealed by the distribution of the Fosbury retrotransposon", Fungal Genetics and Biology 20 (1), Seiten 59-69. Weitere Beispiele für solche Sequenzen lassen sich auf der "Medline"-Website des "National Center for Biotechnology Information" (NCBI) http://www.ncbi.nlm.nih.gov/ unter dem Stichwort LTR finden.

Bei einem weiteren alternativen Verfahren wird im Rahmen der PCR als Vorwärts-Primer ein solcher gewählt, der an eine Nucleotidsequenz eines transposablen Elements auf dem ersten DNA-Strang des Organismus hybridisiert, und als Rückwärts-Primer wird ein solcher gewählt, der an eine Nucleotidsequenz eines transposablen Elements auf dem zu dem ersten DNA-Strang komplementären DNA-Strang des Organismus hybridisiert.

Transposable oder transponierbare Elemente sind mobile genetische Elemente, d.h. DNA-Bereiche, die ihre Position innerhalb des Genoms ändern können. Die Erfinder haben erkannt, dass solche transposablen Elemente ähnlich wie bspw. Satelliten-DNA über das Genom verteilt vorliegen und dazwischenliegende Genomabschnitte meist intergenisch und hochvariabel zwischen Organismen bzw. Individuen sind. Auch transposable Elemente, die gleichzeitig auf Virusgenom zurückführbare Sequenzen darstellen können, sind im Stand der Technik beschrieben; vgl. vgl. Cooper und Geoffrey (a.a.O.); Brown, T. A. (a.a.O.); Pearson und Morrow (a.a.O.); Bernardi, G. (a.a.O.) und Baltimore, D. (a.a.O.).

In einem weiteren alternativen Verfahren wird im Rahmen der PCR als Vorwärts-Primer ein solcher gewählt, der an eine repetitive Nucleotidsequenz eines Telomers oder Centromers auf dem ersten DNA-Strang des Organismus hybridisiert, und als Rückwärts-Primer wird ein solcher gewählt, der an eine repetitive Nucleotidsequenz eines Telomers oder Centromers auf dem zu dem ersten DNA-Strang komplementären DNA-Strang des Organismus hybridisiert.

Auch mit dieser Maßnahme gelingt es nach Erkenntnissen der Erfinder, einen genetischen Fingerabdruck von der genetischen Information des Organismus in Form eines Musters von Amplifikaten von Genomfragmenten zu erstellen. So ist es bekannt, dass in den Centromer- und Telomerbereichen von Chromosomen Wiederholungen von Hunderten oder Tausenden hintereinandergeschalteten DNA-Abschnitten liegen, die in ihrem Aufbau dem von Satelliten-DNA entsprechen. Diese repetitiven Nucleotidsequenzen weisen ebenfalls eine sehr hohe Variabilität zwischen Individuen auf und eignen sich deshalb gleichermaßen zur Entwicklung von hochspezifischen PCR-Sonden und Primern zum Nachweis dieser Individuen bzw. Organismen. Repetitive Nucleotidsequenzen in den Centromer- und Telomerbereichen von Chromosomen sind im Stand der Technik umfassend beschrieben; vgl. Brown, T.A. (a.a.O.); Griffiths, et al. (1999), "Modern genetic analysis", New York: W. H. Freeman & Co.; Cooper und Geoffrey (a.a.O.) und Alberts et al. (2002), "Molecular biology of the cell" 4. Auflage, New York and London: Garland Science. In dem beigefügten Sequenzprotokoll wird ein Beispiel zu einer solchen Sequenz unter SEQ ID-Nr. 38 gegeben, das aus Borovkov und McClean (1993), "A tandemly repeated sequence from the Plasmopara halstedii genome", Gene 124 (1), Seiten 127-130 stammt und von den Erfindern bereits erfolgreich genutzt wurde, um ein organismusspezifisches hybridisierbares Nucleinsäuremolekül herzustellen, das geeignet ist *Plasmopara halstedii* mit hoher Sensitivität und Spezifität nachzuweisen (siehe auch das Ausführungsbeispiel).

Die Erfinder haben erkannt, dass Fragmente von genomischer Information, die mittels eines DNA-Fingerprintings von Organismen gewonnen werden können, eine sehr hohe Variabilität nicht nur zwischen Organismen verschiedener Spezies sondern auch zwischen *forma specialis* und sogar Individuen aufweisen und sich deshalb zur Herstellung von organismusspezifischen hybridisierbaren Nucleinsäuremolekülen, bspw. in Form von PCR-Primern und/oder Hybridisierungssonden, besonders eignen. Diese Fragmente liegen i.d.R. zwischen repetitiven DNA-Sequenzen, die über das Genom des Mikroorganismus hinweg statistisch verteilt sind, wie bspw. zwischen Satelliten-DNA oder auch zwischen solchen auf ein Virus-Genom zurückführbaren Nucleotidsequenzen, zwischen transposablen Elementen oder zwischen repetitiven Nucleotidsequenzen eines Telomers oder Centromers. Auch Nucleotidsequenzen die zwischen randomisierten kurzen Nucleotidsequenzen von 3 bis 30 Nucleotiden liegen, können zu diesem Zweck genutzt werden.

Vor diesem Hintergrund betrifft die vorliegende Erfindung auch die Verwendung eines mit den Merkmalen des Patentanspruchs 7.

"Zwischen zwei Elementen auf der genetischen Information eines Organismus" bedeutet erfindungsgemäß, dass die in Rede stehende Nucleotidsequenz 5'- und 3'-wärts von den beiden Elementen flankiert wird, d.h. an das 3'-Ende der Nucleotidsequenz schließt sich das eine Element, and das 3'-Ende schließt sich das andere Element an.

Im beiliegenden Sequenzprotokoll sind Beispiele für Nucleotidsequenzen solcher Nucleinsäuremoleküle genannt. Diese Nucleinsäuremoleküle leiten sich aus den Genomen der Pflanzenpathogene *Peronospora arborescens*, *Peronospora swinglei* s.l., *Fusarium oxysporum, Plasmopara petroselini* und *Peronospora valerianellae* ab und werden sowohl 5'- als auch 3'-wärts von Mikrosatelliten oder Single Sequence Repeats (SSRs) flankiert. Diese beispielhaften Nucleotidsequenzen sind im beiliegenden Sequenzprotokoll unter SEQ ID-Nr. 1 bis 8 gelistet; vgl. auch Tabelle 1.

Vor diesem Hintergrund ist Gegenstand der vorliegenden Offenbarung auch ein Nucleinsäuremolekül, das eine der Nucleotidsequenzen SEQ ID Nr. 1 bis 8 aus dem beiliegenden Sequenzprotokoll oder eine hierzu komplementäre Nucleotidsequenz aufweist.

Die genannten Nucleinsäuremoleküle haben sich als besonders geeignet erwiesen, um als Matrize für die Herstellung von hybridisierbaren Nucleinsäuremolekülen zu fungieren, mit denen die Organismen *Peronospora arborescens*, *Peronospora swinglei* s.l., *Fusarium oxysporum*, *Plasmopara petroselini* sowie *Peronospora valerianellae* in einer biologischen Probe nachgewiesen werden können.

Dabei können die vorstehend genannten Nucleinsäuremoleküle eine Nucleotidsequenz von 3 bis 812, vorzugsweise von 10 bis 64, weiter vorzugsweise 12 bis 48, weiter vorzugsweise 14 bis 36, weiter vorzugsweise 16 bis 27 aufeinanderfolgenden Nucleotiden aus einer der Nucleoridsequenzen SEQ ID Nr. 1 bis 8 oder einer hierzu komplementäre Nucleotidsequenz aufweisen.

Mit dieser Maßnahme werden bereits Nucleinsäuremoleküle bereit gestellt, die einen Abschnitt mit einer ausreichenden Länge aufweisen, um als PCR-Primer für den Nachweis der Organismen *Peronospora arborescens*, *Peronospora swinglei* s.l., *Fusarium oxysporum, Plasmopara petroselini* sowie *Peronospora valerianellae* zu fungieren. Diese PCR-Primer sind in der Lage an solche Nucleinsäuremoleküle zu hybridisieren, die eine der Nucleotidsequenzen SEQ ID Nr. 1 bis 8 oder eine hierzu komplementäre Sequenz aufweisen.

Ein weiterer Gegenstand der vorliegenden Offenbarung betrifft ein Nucleinsäuremolekül, das zumindest 20 %, bevorzugt zumindest 30 %, weiter bevorzugt 40 %, vorzugsweise 50 %, weiter vorzugsweise 60 %, weiter vorzugsweise 70 %, weiter vorzugsweise 80 %, weiter vorzugsweise 90 %, weiter vorzugsweise 95 %, weiter vorzugsweise 99 % Sequenzhomologie mit den vorstehenden Nucleinsäuremolekülen aufweist.

Auch mit solchen Nucleinsäuremolekülen können aufgrund der hohen Homologie hochselektiv und spezifisch in einer biologischen Probe die drei genannten pflanzenpathogenen Mikroorganismen nachgewiesen werden. Die Homologien lassen sich leicht mittels dem Fachmann bekannter Verfahren ermitteln, bspw. einer BLAST-Analyse oder mittels des MegAlign Moduls des Lasergene Programms von DNAStar Inc. Die angegebenen Identitäten beziehen sich auf das gesamte, vorstehend genannte, erfindungsgemäße Nucleinsäuremolekül bzw. auf einen Abschnitt hiervon mit ≥ 10 Nucleotiden.

Offenbart ist ferner ein solches Nucleinsäuremolekül, das unter stringenten Bedingungen an eines der vorstehend genannten Nucleinsäuremoleküle hybridisiert.

Auch ein solches Nucleinsäuremolekül weist die Eigenschaften der zuvor beschriebenen Nucleinsäuremoleküle auf und ist deshalb zum spezifischen Nachweis der Pflanzenpathogene geeignet. Unter stringenten Bedingungen werden solche verstanden, unter denen nur nahezu perfekt, d.h. zu 80 %, 85 %, 90 %, 95 %, 98 %, 99 % basengepaarte Nucleinsäurestränge gebildet werden und stabil bleiben. Stringente Bedingungen lassen sich bspw. durch die Veränderung der Salzkonzentrationen, dem pH-Wert, der Temperatur etc. in dem Medium einstellen, in dem die Bindung der Nucleinsäurestränge erfolgen soll.

Weitere Kriterien, die der Fachmann bei der Ausgestaltung von PCR-Primern berücksichtigen kann, finden sich weiter oben im Zusammenhang mit der Erläuterung des Schrittes 5 des erfindungsgemäßen Verfahrens sowie in Sambrook und Russel (a.a.O.), Chapter 8, Table 8.3.

Offenbart ist ausserdem ein Verfahren zur Herstellung eines organismusspezifischen hybridisierbaren Nucleinsäuremoleküls, das folgende Schritte aufweist: (1) Bereitstellung einer aus einem Organismus stammenden genomischen Information; (2) Inkubation der genomischen Information des Organismus mit zumindest einer Restriktionsendonuclease zum Erhalt einer Mischung von Restriktionsfragmenten; (3) Ligation von Oligonucleotiden einer definierten Nucleotidsequenz (Adaptorsequenz) an die freien Enden der Restriktionsfragmente zum Erhalt von modifizierten Abschnitten der genomischen Information; (4) Erstellung eines genetischen Fingerabdrucks von der genomischen Information zum Erhalt eines Musters von Amplifikaten von Genomabschnitten durch folgende Schritte: (4.1) Amplifizierung der Fragmente der genomischen Information des Organismus zum Erhalt einer Mischung von Amplifikaten, (4.2) Vereinzelung der in der Mischung enthaltenen Amplifikate zum Erhalt eines Musters von Amplifikaten von Genomabschnitten; (5) Isolierung von zumindest einem Amplifikat aus dem Muster; (6) Sequenzierung des isolierten Amplifikats zum Erhalt einer Nucleotidsequenz; (7) Auswahl eines Abschnitts der Nucleotidsequenz von 3 bis 1000 Nucleotiden, der zur Herstellung eines organismusspezifischen hybridisierbaren Nucleinsäuremoleküls geeignet ist, und (8) Herstellung eines Nucleinsäuremoleküls, das den in Schritt (6) ausgewählten Abschnitt aufweist, wobei die Amplifizierung in Schritt 4.1 mittels Polymerase-Kettenreaktion (PCR) erfolgt und als Vorwärts-Primer ein solcher gewählt wird, der an die Adaptorsequenz auf dem ersten DNA-Strang des Organismus hybridisiert, und als Rückwärts-PCR-Primer ein solcher gewählt wird, der an die Adaptorsequenz auf dem zu dem ersten DNA-Strang komplementären DNA-Strang des Organismus hybridisiert.

Bei diesem Verfahren wird im Rahmen des DNA-Fingerprintings eine etablierte Methode mit der Bezeichnung "amplified fragment length polymorphism" (AFLP) verwendet, die ebenfalls zum Erhalt eines Musters von Amplifikaten von Genomfragmenten mit gewünschten Eigenschaften führt. Im ersten Schritt wird die genomische Information des interessierenden Organismus mit einer Restriktionsendonuclease mit bekannter Schnittstellenspezifität geschnitten. Im nächsten Schritt werden kurze Nucleinsäuremoleküle als sog. Adaptoren bereitgestellt, die durch den Fachmann derart ausgestaltet werden, dass sie komplementär zu den Enden der generierten Restriktionsfragmente sind und ferner Hybridisierungsstellen für PCR-Primer für den folgenden PCR-Schritt aufweisen. Die Adaptoren weisen demnach eine dem Fachmann bekannte Sequenz auf, die sich an der Restriktionsschnittstelle und der Sequenz der PCR-Primer für die anschließende Amplifizierung orientiert. Im nächsten Schritt erfolgt eine Amplifizierung der Ligationsprodukte bzw. der genomischen Fragmente mittels Polymerase-Kettenreaktion (PCR). Dazu werden Primer verwendet, die zu den Adaptoren passen. Als Amplifikat wird dann das zwischen den Adaptoren liegende Genomfragment erhalten. Fakultativ können Maßnahmen zur Reduzierung der Anzahl der genomischen Fragmente ergriffen werden, sollte diese aufgrund der gewählten Restriktionsendonuclease besonders hoch und damit nur schlecht handhabbar sein. Hierfür können bspw. weitere Amplifizierungen nachgeschaltet werden, was zu einer Reduzierung der genomischen Fragmente führt. Das AFLP-Verfahren ist bspw. beschrieben in Weising et al. (a.a.O.).

Offenbart ist ferner ein Verfahren zur Herstellung eines organismusspezifischen hybridisierbaren Nucleinsäuremoleküls, das folgende Schritte aufweist: (1) Bereitstellung einer aus einem Organismus stammenden genomischen Information; (2) Erstellung eines genetischen Fingerabdrucks von der genomischen Information zum Erhalt eines Musters von Amplifikaten von Genomabschnitten durch folgende Schritte: (2.1) Inkubation der genomischen Information mit zumindest einer Restriktionsendonuclease zum Erhalt einer Mischung von Fragmenten der genomischen Information, (2.2) Ligation der Fragmente der genomischen Information in Vektoren, (2.3) Einbringung der Vektoren in Wirtsorganismen, fakultativ (2.3') Ausplattierung der Wirtsorganismen auf Kultivierungsplatten, (2.4) Amplifizierung der Vektoren in den Wirtsorganismen, (2.5) Isolierung der amplifizierten Vektoren zum Erhalt einer Mischung von Amplifikaten von Genomabschnitten, und (2.6) Vereinzelung der in der Mischung enthaltenen Amplifikate zum Erhalt eines Musters von Amplifikaten von Genomabschnitten; (3) Isolierung von zumindest einem Amplifikat aus dem Muster; (4) Sequenzierung des isolierten Amplifikats zum Erhalt einer Nucleotidsequenz; (5) Auswahl eines Abschnitts der Nucleotidsequenz von 3 bis 1000 Nucleotiden, der zur Herstellung eines organismusspezifischen hybridisierbaren Nucleinsäuremoleküls geeignet ist, und (6) Herstellung eines Nucleinsäuremoleküls, das den in Schritt (5) ausgewählten Abschnitt aufweist.

Dieses Verfahren stellt nach Erkenntnissen der Erfinder eine alternative Vorgehensweise zur Erstellung eines genetischen Fingerabdrucks dar, das mittels laborüblicher Reagenzien und Methoden zuverlässig durchgeführt werden kann.

Bei dem erfindungsgemäßen Verfahren erfolgt die Vereinzelung der in der Mischung enthaltenen Amplifikate bspw. mittels einer chromatographischen und/oder elektrophoretischen Methode.

Besonders geeignet sind gelelektrophoretische Verfahren, bei denen die Amplifikate durch die Wanderung in einem elektrischen Feld bspw. entsprechend ihrer Größe voneinander getrennt werden. Geeignet sind jedoch auch chromatographische Verfahren, wie die Hochleistungs-Flüssigkeits-Chromatographie (HPLC), sowie sämtliche Verfahren, die zur Trennung von in einer Mischung vorliegenden Nucleinsäuremolekülen unterschiedlicher Zusammensetzung und/oder Länge und/oder Ladung geeignet sind. Derartige Verfahren sind dem Fachmann allgemein bekannt; vgl. Sambrook und Russell (a.a.O.).

Bei dem erfindungsgemäßen Verfahren wird aus dem Muster von Amplifikaten von Genomabschnitten zumindest ein solches Amplifikat isoliert, das in Relation zu den übrigen Amplifikaten überdurchschnittlich stark amplifiziert wurde.

Wie die Erfinder feststellen konnten, eignen sich solche Amplifikate, die in hoher Menge vorliegen, besonders gut als Matrize zur Herstellung eines speziespezifischen hybridisierbaren Nucleinsäuremoleküls. Die solchen Amplifikaten zugrunde liegenden Abschnitte liegen in der genomischen Information bzw. dem Genom des interessierenden Organismus entweder in hoher Kopienzahl vor oder bilden bei der Amplifizierung kaum störende Sekundärstrukturen aus. Solche besonders gut amplifizierbare Abschnitte lassen sich bspw. nach gelelektrophoretischer Auftrennung der Mischung von Amplifikaten und anschließender Anfärbung mit einer geeigneten Substanz, bspw. Ethidiumbromid, anhand einer besonders stark ausgeprägten bzw. prominenten Gelbande erkennen. Die Gelbanden können bspw. densidometrisch vermessen und es kann ein Durchschnitt der Messwerte gebildet werden. Eine "überdurchschnittlich" starke Amplifizierung eines Amplifikates liegt vor, wenn einer Gelbande in der Densidometrie überdurchschnittliche Werte zugeordnet werden können. Besonders bevorzugt ist es, wenn ein überdurchschnittlich amplifiziertes Amplifikat doppelt, dreifach, vierfach, fünffach, oder zehnfach so stark amplifiziert wird, wie der Durchschnitt der Amplifikate. Die Isolierung eines solchen überdurchschnittlich stark amplifizierten Amplifikates erfolgt bspw. dadurch, dass die entsprechende Bande aus dem Gel ausgeschnitten wird und das darin enthaltene Amplifikat mittels im Stand der Technik bekannter Verfahren eluiert wird.

In dem erfindungsgemäßen Verfahren ist es ferner bevorzugt, wenn nach der Isolierung von zumindest einem Amplifikat aus dem Muster und vor der Sequenzierung des isolierten Amplifikats folgende weitere Schritte erfolgen: a) Einfügung des isolierten Amplifikats in einen Vektor, b) Einbringen des Vektors in einen geeigneten Organismus, und c) Isolieren des Vektors aus dem Organismus zum Erhalt von zumindest einem isolierten Amplifikat.

Durch diese Maßnahmen werden Ansatzstellen für die anschließende Sequenzierung geschaffen. Die Organismen, vorzugsweise Mikroorganismen wie *Escherichia coli*, "reparieren" den Vektor und machen damit das Amplifikat besonders gut sequenzierbar. Das im Vektor enthaltene Amplifikat steht dadurch für die anschließenden Verfahrensschritte in optimaler Form zur Verfügung.

Der zur Herstellung eines organismusspezifischen hybridisierbaren Nucleinsäuremoleküls ausgewählte Abschnitt weist vorzugsweise eine Länge von 10 bis 64, weiter vorzugsweise 12 bis 48, weiter vorzugsweise 14 bis 36, weiter vorzugsweise 16 bis 27 Nucleotide auf, wobei weiter bevorzugt das im anschließenden in Schritt hergestellte Nucleinsäuremolekül als PCR-Primer oder alternativ als Hybridisierungssonde ausgestaltet wird.

Erfahrungsgemäß weisen Hybridisierungssonden bzw. PCR-Primer die vorstehend genannte Anzahl von Nucleotiden auf, die in ihrer Sequenz komplementär zu der nachzuweisenden Zielsequenz sind. Hybridisierungssonden und PCR-Primer können eine Länge von 5, 10, 15, 20, 30, 50, 100, 150 bzw. 200 bis 2000 Nucleotide aufweisen, und deren Sequenz komplementär zu der Zielsequenz ist. Optimalerweise liegt die Anzahl der Nucleotide bei 20 ± 10. Kriterien, die der Fachmann bei der Ausgestaltung von PCR-Primem berücksichtigt, finden sich in Sambrook und Russel (a.a.O.), Chapter 8, Table 8.3.

Die Erfinder konnten mit dem erfindungsgemäßen Verfahren bereits eine Vielzahl von organismusspezifischen hybridisierbaren Nucleinsäuremolekülen herstellen, die als PCR-Primer verwendet werden können und in dem beigefügten Sequenzprotokoll unter SEQ ID-Nr. 9 bis 35 aufgeführt sind; vgl. auch Tabelle 2.

Gegenstand der vorliegenden Erfindung ist deshalb ferner ein Nucleinsäuremolekül, das eine der Nucleotidsequenzen SEQ ID Nr. 9 bis 35 aus dem beiliegenden Sequenzprotokoll oder eine hierzu komplementäre Nucleotidsequenz aufweist.

Ein weiterer Gegenstand der vorliegenden Erfindung betrifft ein Nucleinsäuremolekül, das zumindest 20 %, bevorzugt zumindest 30 %, weiter bevorzugt 40 %, vorzugsweise 50 %, weiter vorzugsweise 60 %, weiter vorzugsweise 70 %, weiter vorzugsweise 80 %, weiter vorzugsweise 90 %, weiter vorzugsweise 95 %, weiter vorzugsweise 99 % Sequenzhomologie mit den vorstehenden Nucleinsäuremolekülen aufweist.

Auch mit solchen Nucleinsäuremolekülen kann aufgrund der hohen Homologie hochselektiv und speziesspezifisch ein pflanzenpathogener Mikroorganismus nachgewiesen werden. Die Homologien lassen sich leicht mittels dem Fachmann bekannter Verfahren ermitteln, bspw. einer BLAST-Analyse oder mittels des MegAlign Moduls des Lasergene Programms von DNAStar Inc. Die angegebenen Identitäten beziehen sich auf die gesamten vorstehend genannten erfindungsgemäßen Nucleinsäuremoleküle bzw. auf einen Abschnitt hiervon mit ≥ 10 Nucleotiden.

Ein weiterer Gegenstand der vorliegenden Offenbarung betrifft ein Nucleinsäuremolekül, das unter stringenten Bedingungen an eines der zuvor beschriebenen Nucleinsäuremoleküle hybridisiert.

Auch ein solches Nucleinsäuremolekül weist die Eigenschaften der zuvor beschriebenen Nucleinsäuremoleküle auf und ist deshalb zum spezifischen Nachweis der genannten Mikroorganismen geeignet. Bezüglich der Bestimmung von stringenten Bedingungen wird auf die weiter oben gegebene Definition verwiesen.

Ein weiterer Gegenstand der vorliegenden Offenbarung betrifft ein Kit, das zumindest eines der zuvor genannten Nucleinsäuremoleküle, eine Beschreibung zur Verwendung des Nucleinsäuremoleküls, vorzugsweise im Rahmen des nachfolgenden erfindungsgemäßen Nachweisverfahrens, sowie ggf. Puffer und Chemikalien aufweist.

Derartige Kits können bspw. für jeden nachzuweisenden Mikroorganismus als Diagnosekit zusammengestellt werden. Ein solches Diagnosekit enthält dann bspw. die für den jeweiligen nachzuweisenden pathogenen Organismus hochselektiven PCR-Primer sowie eine DNA-Probe des entsprechenden Organismus als Positivkontrolle. Ferner enthält ein solches Diagnosekit eine Beschreibung mit genauen Informationen dazu, wie das anschließende Nachweisverfahren durchzuführen ist. Es versteht sich, dass das Kit für die Durchführung des Diagnoseverfahrens geeignete Puffer, Chemikalien und sonstige Hilfsstoffe enthalten kann. Die Bereitstellung eines solchen Kits hat den Vorteil, dass alle erforderlichen Chemikalien und Informationen bereitgestellt werden, so dass auch von ggf. wenig geschultem Personal ein entsprechendes Nachweisverfahren fehlerfrei durchgeführt werden kann.

Offenbart ist schliesslich ein Verfahren zum Nachweis eines Organismus in einer biologischen Probe, bspw. einer Saatgutprobe, das folgende Schritte aufweist: 1. Bereitstellung einer Nucleinsäure enthaltenden biologischen Probe, 2. Isolierung der Nucleinsäure aus der Probe, 3. Unterziehung der isolierten Nucleinsäure einer Polymerase-Kettenreaktion (PCR), 4. Feststellung, ob in Schritt 3 eine Amplifizierung der isolierten Nucleinsäure erfolgt ist, und 5. Korrelation einer positiven Feststellung in Schritt 4 mit dem Vorhandensein des Organismus in der Probe, und Korrelation einer negativen Feststellung in Schritt 4 mit der Abwesenheit des Organismus in der Probe, wobei in Schritt 3 als PCR-Primer zumindest ein Nucleinsäuremolekül verwendet wird, das nach dem erfindungsgemäßen Verfahren hergestellt wurde.

Für das vorstehend beschriebene Verfahren gelten die zuvor dargelegten Ausführungen im Zusammenhang mit den übrigen erfindungsgemäßen Gegenständen entsprechend. Es wird hierauf explizit verwiesen.

Die PCR-Reaktion wird vorzugsweise als "nested" oder verschachtelte PCR-Reaktion ausgestaltet.

Diese Maßnahme hat den besonderen Vorteil, dass die Spezifität und Effizienz der durchgeführten PCR weiter erhöht wird. Dabei wird in einer ersten PCR-Runde im Rahmen der sog. Pre-PCR ein erstes Template über wenige Zyklen amplifiziert. Die Primer werden hier so gewählt, dass diese relativ weit auseinander liegen, d.h. der Vorwärts-PCR-Primer hybridisiert beispielsweise relativ nah an dem 3'-Ende, der Antisense-PCR-Primer hybridisiert hingegen beispielsweise nahe an dem 5'-Ende des intergenischen organismusspezifischen DNA-Abschnittes. Das Amplifikat dieser Pre-PCR wird dann im Rahmen einer weiteren PCR-Runde, der Sekundär-PCR oder Post-PCR, unter Verwendung eines neuen PCR-Primerpaares amplifiziert. Die neuen PCR-Primer liegen weiter innen als die ersten, so dass in diesem zweiten Schritt nur noch die spezifischen DNA-Abschnitte aus der Pre-PCR amplifiziert werden. Dadurch wird die Empfindlichkeit und Effizienz des erfindungsgemäßen Verfahrens deutlich erhöht.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung werden nachfolgend näher erläutert, aus denen sich weitere Eigenschaften und Merkmale ergeben. Die Ausführungsbeispiele schränken die Reichweite der Erfindung jedoch keinesfalls ein. Dabei wird Bezug auf die beiliegenden Figuren genommen, in denen Folgendes dargestellt ist:
- Fig. 1: zeigt einen klassischen DNA-Fingerprint zur Durchführung eines Mustervergleichs nach dem Stand der Technik, um den Erreger *Xanthomonas campestris* in Feldproben von Kohlrabi zu identifizieren.;
- Fig. 2: zeigt eine schematische Darstellung des Vorkommens von organismenspezifischen DNA-Sequenzen mit flankierenden repetitiven DNA-Sequenzen am Beispiel eines eukaryotischen Chromosoms;
- Fig. 3: zeigt eine schematische Darstellung wichtiger Schritte des erfindungsgemäßen Verfahrens zur Herstellung von organismusspezifischen Nucleinsäuremolekülen.
- Fig. 4: zeigt das Ergebnis des erfindungsgemäßen Nachweisverfahrens anhand eines mittels Ethidiumbromid angefärbten Agarosegels, um den Erreger *Peronospora swinglei* s.l. in Basilikumsaatgut nachzuweisen.

Die Offenbarüng betrifft auch die folgenden Aspekte, die im Einklang mit der Entscheidung J15/88 der Juristischen Beschwerdekammer des Europäischen Patentamtes in den folgenden Absätzen definiert sind, welche Teil der vorliegenden Beschreibung, nicht aber die Patentansprüche darstellen.
1. Verfahren zur Herstellung eines organismusspezifischen hybridisierbaren Nucleinsäuremoleküls, das folgende Schritte aufweist:
   1. Bereitstellung einer aus einem Organismus stammenden genomischen Information;
   2. Erstellung eines genetischen Fingerabdrucks von der genomischen Information zum Erhalt eines Musters von Amplifikaten von Genomabschnitten durch folgende Schritte:
      2.1 Amplifizierung von Abschnitten der genomischen Information des Organismus zum Erhalt einer Mischung von Amplifikaten, und
      2.2 Vereinzelung der in der Mischung enthaltenen Amplifikate zum Erhalt eines Musters von Amplifikaten von Genomabschnitten;
   3. Isolierung von zumindest einem Amplifikat aus dem Muster;
   4. Sequenzierung des isolierten Amplifikats zum Erhalt einer Nucleotidsequenz;
   5. Auswahl eines Abschnitts der Nucleotidsequenz von 3 bis 1000 Nucleotiden, der zur Herstellung eines organismusspezifischen hybridisierbaren Nucleinsäuremoleküls geeignet ist, und
   6. Herstellung eines Nucleinsäuremoleküls, das den in Schritt (5) ausgewählten Abschnitt aufweist.
2. Verfahren nach Absatz 1, dadurch gekennzeichnet, dass die genomische Information des Organismus doppelsträngige DNA ist.
3. Verfahren nach Absatz 1 oder 2, dadurch gekennzeichnet, dass die Amplifizierung in Schritt 2.1 mittels Polymerase-Kettenreaktion (PCR) erfolgt.
4. Verfahren nach Absatz 3, dadurch gekennzeichnet, dass im Rahmen der PCR als Vorwärts-Primer ein solcher gewählt wird, der an eine Nucleotidsequenz einer Satelliten-DNA auf dem ersten DNA-Strang des Organismus hybridisiert, und als Rückwärts-Primer ein solcher gewählt wird, der an eine Nucleotidsequenz einer Satelliten-DNA auf dem zu dem ersten DNA-Strang komplementären DNA-Strang des Organismus hybridisiert.
5. Verfahren nach Absatz 4, dadurch gekennzeichnet, dass die Satelliten-DNA ausgewählt ist aus der Gruppe bestehend aus: Mikrosatelliten-DNA, simple sequence repeats (SSRs) und intersimple sequence repeats (ISSRs).
6. Verfahren nach Absatz 3, dadurch gekennzeichnet, dass im Rahmen der PCR als Vorwärts-Primer ein solcher gewählt wird, der an eine randomisierte Nucleotidsequenz mit einer Länge von 3 bis 30, vorzugsweise von 4 bis 20, höchst bevorzugt von 6 bis 10 Nucleotiden, auf dem ersten DNA-Strang des Organismus hybridisiert, und als Rückwärts-Primer ein solcher gewählt wird, der an eine Nucleotidsequenz mit einer Länge von 3 bis 30, vorzugsweise von 4 bis 20, höchst bevorzugt von 6 bis 10 Nucleotiden, auf dem zu dem ersten DNA-Strang komplementären DNA-Strang des Organismus hybridisiert.
7. Verfahren nach Absatz 3, dadurch gekennzeichnet, dass im Rahmen der PCR als Vorwärts-Primer ein solcher gewählt wird, der an eine auf ein Virusgenom zurückführbare Nucleotidsequenz auf dem ersten DNA-Strang des Organismus hybridisiert, und als Rückwärts-Primer ein solcher gewählt wird, der an eine auf ein Virusgenom zurückführbare Nucleotidsequenz auf dem zu dem ersten DNA-Strang komplementären DNA-Strang des Organismus hybridisiert.
8. Verfahren nach Absatz 3, dadurch gekennzeichnet, dass im Rahmen der PCR als Vorwärts-Primer ein solcher gewählt wird, der an eine Nucleotidsequenz eines transposablen Elements auf dem ersten DNA-Strang des Organismus hybridisiert, und als Rückwärts-Primer ein solcher gewählt wird, der an eine Nucleotidsequenz eines transposablen Elements auf dem zu dem ersten DNA-Strang komplementären DNA-Strang des Organismus hybridisiert.
9. Verfahren nach Absatz 3, dadurch gekennzeichnet, dass im Rahmen der PCR als Vorwärts-Primer ein solcher gewählt wird, der an eine repetitive Nucleotidsequenz eines Telomers oder Centromers auf dem ersten DNA-Strang des Organismus hybridisiert, und als Rückwärts-Primer ein solcher gewählt wird, der an eine repetitive Nucleotidsequenz eines Telomers oder Centromers auf dem zu dem ersten DNA-Strang komplementären DNA-Strang des Organismus hybridisiert.
10. Verfahren zur Herstellung eines organismusspezifischen hybridisierbaren Nucleinsäuremoleküls, das folgende Schritte aufweist:
   1. Bereitstellung einer aus einem Organismus stammenden genomischen Information;
   2. Inkubation der genomischen Information des Organismus mit zumindest einer Restriktionsendonuclease zum Erhalt einer Mischung von Restriktionsfragmenten;
   3. Ligation von Oligonucleotiden einer definierten Nucleotidsequenz (Adaptorsequenz) an die freien Enden der Restriktionsfragmente zum Erhalt von modifizierten Abschnitten der genomischen Information;
   4. Erstellung eines genetischen Fingerabdrucks von der genomischen Information zum Erhalt eines Musters von Amplifikaten von Genomabschnitten durch folgende Schritte:
      4.1 Amplifizierung der modifizierten Abschnitte der genomischen Information des Organismus zum Erhalt einer Mischung von Amplifikaten,
      4.2 Vereinzelung der in der Mischung enthaltenen Amplifikate zum Erhalt eines Musters von Amplifikaten von Genomabschnitten;
   5. Isolierung von zumindest einem Amplifikat aus dem Muster;
   6. Sequenzierung des isolierten Amplifikats zum Erhalt einer Nucleotidsequenz;
   7. Auswahl eines Abschnitts der Nucleotidsequenz von 3 bis 1000 Nucleotiden, der zur Herstellung eines organismusspezifischen hybridisierbaren Nucleinsäuremoleküls geeignet ist, und
   8. Herstellung eines Nucleinsäuremoleküls, das den in Schritt (6) ausgewählten Abschnitt aufweist,
   wobei die Amplifizierung in Schritt 4.1 mittels Polymerase-Kettenraktion (PCR) erfolgt und als Vorwärts-Primer ein solcher gewählt wird, der an die Adaptorsequenz auf dem ersten DNA-Strang des Organismus hybridisiert, und als Rückwärts-PCR-Primer ein solcher gewählt wird, der an die Adaptorsequenz auf dem zu dem ersten DNA-Strang komplementären DNA-Strang des Organismus hybridisiert.
11. Verfahren zur Herstellung eines organismusspezifischen hybridisierbaren Nucleinsäuremoleküls, das folgende Schritte aufweist:
   1. Bereitstellung einer aus einem Organismus stammenden genomischen Information;
   2. Erstellung eines genetischen Fingerabdrucks von der genomischen Information zum Erhalt eines Musters von Amplifikaten von Genomabschnitten durch folgende Schritte:
      2.1 Inkubation der genomischen Information mit zumindest einer Restriktionsendonuclease zum Erhalt einer Mischung von Fragmenten der genomischen Information,
      2.2 Ligation der Fragmente der genomischen Information in Vektoren,
      2.3 Einbringung der Vektoren in Wirtsorganismen,
      2.4 Amplifizierung der Vektoren in den Wirtsorganismen,
      2.5 Isolierung der amplifizierten Vektoren zum Erhalt einer Mischung von Amplifikaten von Genomabschnitten, und
      2.6 Vereinzelung der in der Mischung enthaltenen Amplifikate zum Erhalt eines Musters von Amplifikaten von Genomabschnitten,
   3. Isolierung von zumindest einem Amplifikat aus dem Muster;
   4. Sequenzierung des isolierten Amplifikats zum Erhalt einer Nucleotidsequenz;
   5. Auswahl eines Abschnitts der Nucleotidsequenz von 3 bis 1000 Nucleotiden, der zur Herstellung eines organismusspezifischen hybridisierbaren Nucleinsäuremoleküls geeignet ist, und
   6. Herstellung eines Nucleinsäuremoleküls, das den in Schritt (5) ausgewählten Abschnitt aufweist.
12. Verfahren nach Absatz 11, dadurch gekennzeichnet, dass nach Schritt 2.3 und vor Schritt 2.4 folgender Schritt erfolgt:
   2.3' Ausplattierung der Wirtsorganismen auf Kultivierungsplatten.
13. Verfahren nach einem der Absätze 1 bis 12, dadurch gekennzeichnet, dass die Vereinzelung der in der Mischung enthaltenen Amplifikate mittels einer chromatographischen und/oder elektrophoretischen Methode erfolgt.
14. Verfahren nach einem der vorherigen Absätze, dadurch gekennzeichnet, dass aus dem Muster von Amplifikaten von Fragmenten der genomischen Information ein solches Amplifikat isoliert wird, das in Relation zu den übrigen Amplifikaten bei der Amplifizierung überdurchschnittlich stark amplifiziert wurde.
15. Verfahren nach einem der vorherigen Absätze, dadurch gekennzeichnet, dass nach der Isolierung von zumindest einem Amplifikat aus dem Muster und vor der Sequenzierung des isolierten Amplifikats folgende weitere Schritte erfolgen:
   a) Einfügung des isolierten Amplifikats in einen Vektor,
   b) Einbringen des Vektors in einen geeigneten Organismus, und
   c) Isolieren des Vektors aus dem Organismus zum Erhalt von zumindest einem isolierten Amplifikat.
16. Verfahren nach einem der vorherigen Absätze, dadurch gekennzeichnet, dass der zur Herstellung eines organismusspezifischen hybridisierbaren Nucleinsäuremoleküls geeignete ausgewählte Abschnitt eine Länge von 10 bis 64, vorzugsweise 12 bis 48, weiter vorzugsweise 14 bis 36, weiter vorzugsweise 16 bis 27 Nucleotiden aufweist.
17. Verfahren nach einem der vorherigen Absätze, dadurch gekennzeichnet, dass das hergestellte Nucleinsäuremolekül als PCR-Primer ausgestaltet wird.
18. Nucleinsäuremolekül, das eine der Nucleotidsequenzen SEQ ID Nr. 9 bis 35 aus dem beiliegenden Sequenzprotokoll oder eine hierzu komplementäre Nucleotidsequenz aufweist.
19. Nucleinsäuremolekül, das zumindest 20%, bevorzugt zumindest 30%, weiter bevorzugt 40%, vorzugsweise 50%, weiter vorzugsweise 60%, weiter vorzugsweise 70%, weiter vorzugsweise 80%, weiter vorzugsweise 90%, weiter vorzugsweise 95%, weiter vorzugsweise 99% Sequenzhomologie mit dem Nucleinsäuremolekül, das eine der Nucleotidsequenzen SEQ ID Nr. 9 bis 35 aus dem beiliegenden Sequenzprotokoll oder eine hierzu komplementäre Nucleotidsequenz aufweist, aufweist.
20. Nucleinsäuremolekül, das unter stringenten Bedingungen an das Nucleinsäuremolekül gemäß Absatz 18 oder 19 hybridisiert.
21. Verwendung eines Nucleinsäuremoleküls mit einer Nucleotidsequenz, die zwischen zwei Elementen der genetischen Information eines Organismus liegt, zur Herstellung eines organismenspezifischen hybridisierbaren Nucleinsäuremoleküls, dadurch gekennzeichnet, dass die zwei Elemente jeweils unabhängig voneinander ausgewählt sind aus der Gruppe bestehend aus: Satelliten-DNA-Nucleotidsequenz, auf Virusgenom zurückführbare Nucleotidsequenz, Nucleotidsequenz eines transposablen Elementes, repetitive Nucleotidsequenz eines Telomers/Centromers.
22. Verwendung nach Absatz 21, dadurch gekennzeichnet, dass das organismusspezifische hybridisierbare Nucleinsäuremolekül ein PCR-Primer ist.
23. Nucleinsäuremolekül, das zumindest 20%, bevorzugt zumindest 30%, weiter bevorzugt 40%, vorzugsweise 50%, weiter vorzugsweise 60%, weiter vorzugsweise 70%, weiter vorzugsweise 80%, weiter vorzugsweise 90%, weiter vorzugsweise 95%, weiter vorzugsweise 99% Sequenzhomologie mit dem Nucleinsäuremolekül, das eine der Nucleoridsequenzen SEQ ID Nr. 1 bis 8 aus dem beiliegenden Sequenzprotokoll oder eine hierzu komplementäre Nucleotidsequenz aufweist, aufweist.
24. Nucleinsäuremolekül, dadurch gekennzeichnet, dass es eine Nucleotidsequenz von 3 bis 812, vorzugsweise von 10 bis 64, weiter vorzugsweise 12 bis 48, weiter vorzugsweise 14 bis 36, weiter vorzugsweise 16 bis 27 aufeinanderfolgenden Nucleotiden aus einer der Nucleotidsequenzen SEQ ID Nr. 1 bis 4 oder einer hierzu komplementäre Nucleotidsequenz aufweist.
25. Nucleinsäuremolekül, das eine der Nucleoridsequenzen SEQ ID Nr. 1 bis 4 aus dem beiliegenden Sequenzprotokoll oder eine hierzu komplementäre Nucleotidsequenz aufweist.
26. Nucleinsäuremolekül, das unter stringenten Bedingungen an das Nucleinsäuremolekül gemäß einem der Absätze 23 bis 25 hybridisiert.
27. Kit, das zumindest eines der Nucleinsäuremoleküle gemäß einem der Absätze 18 bis 20 und/oder 23 bis 26, eine Beschreibung zur Durchführung des Verfahrens nach einem der Absätze 28 bis 30 sowie ggf. Puffer und Chemikalien aufweist.
28. Verfahren zum Nachweis eines Organismus in einer biologischen Probe, das folgende Schritte aufweist:
   1. Bereitstellung einer Nucleinsäure enthaltenden biologischen Probe,
   2. Isolierung der Nucleinsäure aus der Probe,
   3. Unterziehung der isolierten Nucleinsäure einer Polymerase-Kettenreaktion (PCR),
   4. Feststellung, ob in Schritt 3 eine Amplifizierung der isolierten Nucleinsäure erfolgt ist, und
   5. Korrelation einer positiven Feststellung in Schritt 4 mit dem Vorhandensein des Organismus in der Probe, und Korrelation einer negativen Feststellung in Schritt 4 mit der Abwesenheit des Organismus in der Probe,
   dadurch gekennzeichnet, dass in Schritt 3 als PCR-Primer zumindest ein Nucleinsäuremolekül verwendet wird, das nach dem Verfahren gemäß einem der Absätze 1 bis 17 hergestellt wurde.
29. Verfahren nach Absatz 32, dadurch gekennzeichnet, das als PCR-Primer zumindest ein Nucleinsäuremolekül nach einem der Absätze 18 bis 20 verwendet wird.
30. Verfahren nach Absatz 28 oder 29, dadurch gekennzeichnet, dass die PCR-Reaktion in Schritt 3 als nested-PCR-Reaktion ausgestaltet ist.

### Ausführungsbeispiele

### Beispiel 1: Nachweis von Xanthomonas campestris über einen "Mustervergleich" (Stand der Technik)

Der bakterielle Erreger *Xanthomonas campestris* pathovar *campestris* ist einer der wichtigsten Schädlinge im Kohlanbau. Zur Identifikation des Organismus wird im Stand der Technik ein Verfahren eingesetzt, das auf dem Vergleich von genetischen Fingerabdrücken basiert ("Mustervergleich"). Die Methode und die damit einhergehenden Probleme sollen im Folgenden an Hand von 9, aus dem Feldanbau von Kohlrabi und verschiedenen Wildpflanzen isolierten Reinkulturen demonstriert werden.

Das Standardprotokoll im Stand der Technik umfasst im Wesentlichen folgende 4 Arbeitsschritte, nämlich (a) die Bereitstellung der Organismen in Form einer Reinkultur, (b) die DNA-Extraktion, (c) die Herstellung eines genetischen Fingerabdrucks und (d) die elektrophoretische Trennung der genetischen Fingerabdrücke

### (a) Bereitstellung der Organismen in Form einer Reinkultur

Die makroskopisch als erkrankt identifizierten Pflanzenteile wurden in ein steriles Reagenzglas überführt, in dem ein Flüssigmedium wie z.B. das NYG-Medium vorgelegt war. Nach einer Inkubationszeit von min. 12 h bei 30°C wurden auf Agarplatten Verdünnungsausstriche mit verschieden konzentrierten Lösungen des Flüssigmediums angefertigt und über Nacht bei 30°C inkubiert. Am anderen Tag wurden vereinzelte Bakterienkolonien von der Agarplatte abgehoben und weitere Verdünnungsausstriche angefertigt. Dieser Prozess wurde so oft wiederholt, bis Gewissheit bestand, dass es sich bei dem Ausstrich tatsächlich um eine Reinkultur des Organismus handelt. Mit diesen Reinkulturen wurden gemäß den Kochschen Postulaten Reinfektionen zur Verifizierung der Pathogenität durchgeführt. Für die weiteren Versuche wurden nur diejenigen Reinkulturen weiter verwendet, die die typischen Infektionssymptome des Erregers aufwiesen.

### (b) DNA-Extraktion

Die Extraktion der bakteriellen DNA wurde mit dem GenElute^{™} Bacterial Genomic DNA Kit (Sigma Aldrich, Deutschland) gemäß den Angaben des Herstellers durchgeführt. Die gewonnene DNA-Lösung wurde anschließend photometrisch auf Quantität und Qualität geprüft.

### (c) Herstellung eines genetischen Fingerabdrucks

Eine PCR-Reaktion wird in einem PCR-geeigneten Reaktionsgefäß angesetzt, bspw. bestehend aus 2,5 µl 10x PCR-Puffer, 2,5 µl einer 2 mM Lösung der vier Desoxynucleotidtriphosphate (dNTPs), 2,5 µl Magnesiumchloridlösung (20 mM), 1 U einer thermostabilen DNA-Polymerase, bspw. Taq DNA-Polymerase, 1 µl einer bspw. 25 mM Lösung des PCR-Primers GTC₅ (5'GTCGTCGTCGTCGTC-3'; SEQ ID-Nr. 39). Der Primer ist so ausgestaltet, dass er an den Mikrosatelliten GTC binden kann. Der Ansatz wird mit Wasser auf 25 µl aufgefüllt. Es erfolgt die Durchführung der PCR, bspw. mit folgenden Bedingungen: Die Inkubation in einem Thermocycler erfolgt gemäß den gerätespezifischen Modalitäten. Zunächst erfolgt bspw. eine Inkubation, bspw. bei 94°C für 2 min. Anschließend erfolgt eine Inkubation bei bspw. 94°C, für 1 min. Danach eine Inkubation, bspw. bei 56°C, für 1 min. Anschließend erfolgt eine Inkubation, bspw. bei 72°C, für 2 min. Die letzten drei Schritte werden anschließend bspw. 30 Mal wiederholt. Es kann dann bspw. eine abschließende Inkubation, bspw. bei 72°C für 4 min, durchgeführt werden.

### (d) Elektrophoretische Trennung der genetischen Fingerabdrücke

Die in der PCR erhaltenen Amplifikate werden aufgetrennt, bspw. mittels Gelelektrophorese, bspw. in einem Agarosegel. Dieses kann bspw. hergestellt werden, indem 1 g Agarose in ein Gefäß gegeben werden, auf 100 pl mit einer ionenhaltigen Flüssigkeit aufgefüllt wird, bspw. mit Tris-Borat-EDTA-Puffer (TBE).

Danach wird die PCR-Reaktionslösung in eine präformierte Vertiefung des Agarosegels gegeben, bspw. nach Versetzen mit 1/6 vol. Glycerin und ggf. Farbstoffen, die bei der Elektrophorese in die gleiche Richtung laufen wie die DNA-Fragmente im Agarosegel und der Kontrolle des Fortschreitens der Elektrophorese dienen. Die Elektrophorese selbst erfolgt in einer dafür vorgesehenen Elektrophoresekammer, die mit einer zum Gel isotonischen ionenhaltigen Lösung gefüllt ist, bevorzugterweise mit derselben, die zur Herstellung des Agarosegels verwandt worden ist. Durch Anlegen einer Spannung, bspw. 140 V, wird die Wanderung der DNA-Fragmente durch das Agarosegel erzwungen, die durch das selektive Rückhaltevermögen, welches insbesondere auf der Größe der wandernden Fragmente beruht, aufgetrennt werden. Anschließend erfolgt eine Färbung mit einem an Nucleinsäuren assoziierenden oder bindenden Farbstoff, bspw. Ethidiumbromid, bspw. durch inkubieren des Agarosegels für eine halbe Stunde in einem ethidiumbromidhaltigen Färbebad.

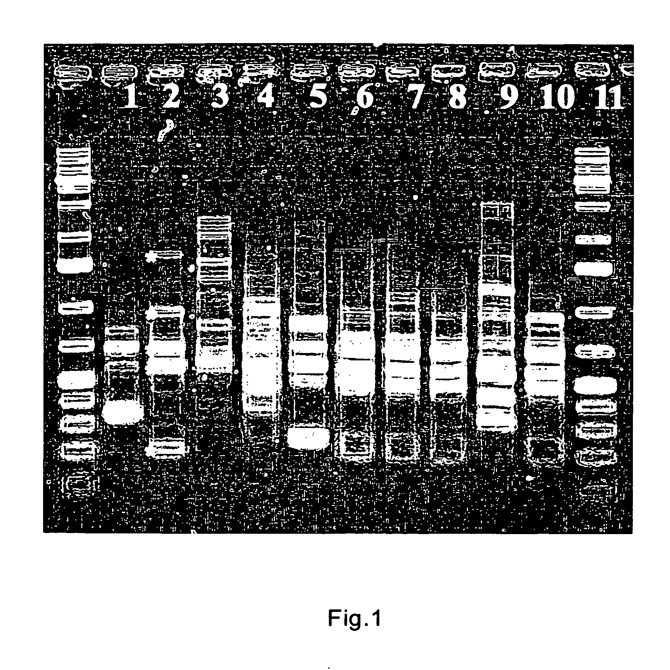

Das Ergebnis dieses Experimentes ist in der Fig. 1 dargestellt: Agarosegel, 1,5 %, gefärbt mit Ethidiumbromid; Eingesetzte Primer: GTC₅Spur 1: *Xanthomonas campestris* Feldprobe 1; Spur 2: *Xanthomonas campestris* Referenzprobe; Spur 3: *Xanthomonas campestris* Feldprobe 2; Spur 4: *Xanthomonas campestris* Feldprobe 3; Spur 5: *Xanthomonas campestris* Feldprobe 4; Spur 6: *Xanthomonas campestris* Feldprobe 5; Spur 7: *Xanthomonas campestris* Feldprobe 6; Spur 8: *Xanthomonas campestris* Feldprobe 7; Spur 9: *Xanthomonas campestris* Feldprobe 8; Spur 10: *Xanthomonas campestris* Feldprobe 9; Spur 11: DNA-Größenmarker (Standard)1

Mit dem eingesetzten Primer GTC₅ konnten für die Referenzprobe von *Xanthomonas campestris* pathovar *campestris* (Spur 2) fünf eindeutige Amplifikate in unterschiedlicher Größe erhalten werden (mit * markiert). Vergleicht man dieses Referenzmuster mit den anderen neun Mustern der zu identifizierenden Organismen, so kann festgestellt werden, dass keines dieser neun Muster mit dem Referenzmuster zu 100 % identisch ist. Eine sichere Identifikation der Organismen kann aufgrund dieser Untersuchung nicht vorgenommen werden.

Das vorgestellte Beispiel zeigt die typischen Nachteile der Identifikation auf Basis eines "Mustervergleichs" von genetischen Fingerabdrücken. Die zu vergleichenden Muster sind meist nicht genau identisch, einzelne Amplifikate sind schwächer oder stärker, Amplifikate fehlen komplett oder es treten neue auf. Um diese Probleme zu minimieren, werden in der Praxis mit derselben DNA-Lösung unterschiedliche Fingerabdrücke, basierend auf unterschiedlichen PCR-Primern, hergestellt. Die einzelnen Vergleichsuntersuchungen werden dann in einem aufwändigen Verfahren miteinander verglichen. Der Aufwand ist groß und für eine schnelle Identifikation, wie sie die landwirtschaftliche Praxis fordert, nicht geeignet. Zudem ist das Ergebnis sehr stark von der eingesetzten Trennmatrix bzw. den Bedingungen der Trennung insgesamt abhängig, was eine Vergleichbarkeit von Testergebnissen zwischen einzelnen Labors sehr erschwert.

### Beispiel 2: Organismenspezifische DNA-Bereiche mit flankierenden repetitiven DNA-Sequenzen

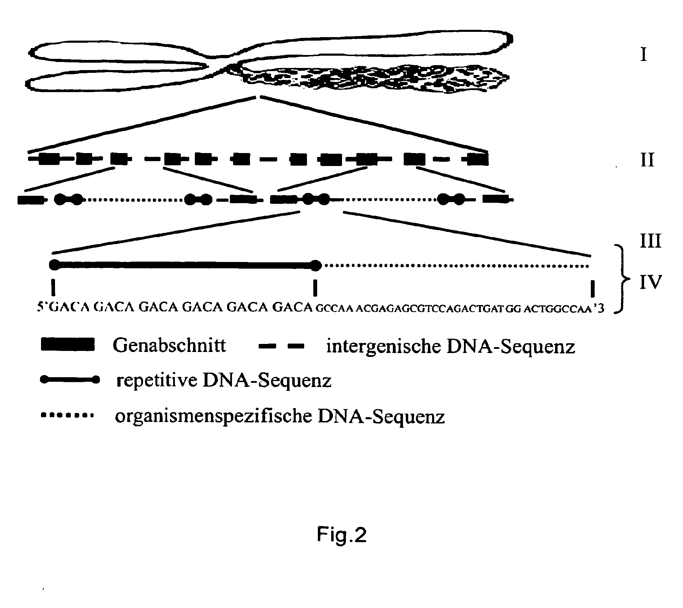

In Fig. 2 ist schematisch ein Teil des Genoms eines Phytopathogens dargestellt. In der Organisationsstufe I ist schematisch ein Chromosom dargestellt; die Organisationsstufe II zeigt den DNA-Doppelstrang, auf dem codierende Genabschnitte und Regionen intergenischer DNA-Sequenzen dargestellt sind; die Organisationsstufe IV stellt ein Sequenzbeispiel für repetitive (z.B. Mikrosatelliten; durchgezogene Linie) und organismenspezifische (gepunktete Linie) DNA-Sequenzen dar.

Die intergenischen Sequenzen einschließlich organismenspezifische DNA-Sequenzen, die zwischen repetitiven Sequenzen liegen bzw. von diesen flankiert werden, eignen sich hervorragend als Matrizen zur Herstellung von organismusspezifischen hybridisierbaren Nucleinsäuremolekülen, wie PCR-Primern, und zwar aus folgenden Gründen: Intergenische Sequenzen sind nicht proteincodierend. (Fig. 2(II)), dadurch ergibt sich ein geringer bis fehlender Selektionsdruck. Sie weisen eine sehr hohe Variabilität zwischen einzelnen Organismen bzw. Spezies auf, haben jedoch eine hohe Spezifität für den jeweiligen Organismus bzw. die jeweilige Spezies. Sie lassen sich durch die flankierenden repetitiven DNA-Sequenzen schnell identifizieren, da sie im Genom weitgehend statistisch verteilt (Fig. 2(II)), jedoch gehäuft in intergenischen Abschnitten zu finden sind (Fig. 2 (III)).

### Beispiel 3: Herstellung eines organismusspezifischen hybridisierbaren Nucleinsäuremoleküls am Beispiel eines PCR-Primers

### 1. Biologische Probe

Als biologische Probe werden Mycel und Sporangien von *Peronospora swinglei* s.l., dem Falschen Mehltau des Basilikums, bereit gestellt. "s.l." bedeutet *sensu lato*, lateinisch für "im weiteren Sinne", da die Identität des Erregers nicht abschließend geklärt ist.

### 2. Isolierung der genomischen Information

5 mg der Sporangien werden in einem Reaktionsgefäß von. 2 ml, mittels eines hierfür geeigneten Gerätes, bspw. einer Schüttelmühle, unter Zugabe von Mahlkörpern, bspw. kleinen Eisenkügelchen, zerkleinert. Die zerkleinerten Sporangien werden mit 500 µl einer Detergens-Lösung versetzt, um die Membranen des Organismus zu zerstören und somit die genomische DNA freizusetzen. Die Lösung kann bspw. bestehen aus 0,1 M Tris pH 8, 0.05 M EDTA, 2% SDS, Proteinase K. Die zerkleinerten Sporangien werden in dem Reaktionsgefäß mit der oben genannten Lösung für mehrere Minuten, bspw. 30 min, bei 65°C, inkubiert. Das Reaktionsgefäß wird bspw. für 5 min bei 10 000 s/t²*9,81 zentrifugiert, um Zelltrümmer zu sedimentieren.

Die flüssige Phase wird abgenommen und in ein neues Reaktionsgefäß überführt. Zu der flüssigen Phase wird eine proteinausfällende, bevorzugterweise eine phasentrennende Lösung, bspw. Chloroform und Isoamylalkohol im Verhältnis 24 zu 1, beispielsweise in gleicher Menge wie die bereits im Reaktionsgefäß befindlichen Flüssigkeit hinzugegeben. Die Phasen werden gemischt, bspw. durch mehrfaches Schwenken des Reaktionsgefäßes entlang der Längsachse. Das Reaktionsgefäß wird gemischt, bspw. für 10 min bei 10 000 s/t²*9,81, um die Phasen der Proteinfällung zu trennen.

Die wässrige Lösung wird in ein neues Reaktionsgefäß überführt. Es erfolgt die Zugabe einer DNA-ausfällenden Lösung, bspw. Isopropanol mit einem Volumen von 50 % des Volumens der überführten wässrigen Phase. Daran an schließt sich eine Inkubation mit der DNA-ausfällenden Lösung, bspw. für 20 min bei 4°C. Das Reaktionsgefäß wird bspw. für 20 min bei 10 000 s/t²*9,81 und 4°C zentrifugiert, um die DNA zu sedimentieren.

Der Überstand wird abgenommen. Die sedimentierte DNA wird getrocknet, bspw. durch Abblasen mit moderatem Luftstrom. Die DNA wird in Pufferlösung oder Wasser, bspw. bidestilliertem Wasser, gelöst.

### 3. Erstellung eines genetischen Fingerabdrucks

Eine PCR-Reaktion wird in einem PCR-geeigneten Reaktionsgefäß angesetzt, bspw. bestehend aus 2,5 µl 10x PCR-Puffer, 2,5 µl einer 2 mM Lösung der vier Desoxynucleotidtriphosphate (dNTPs), 2,5 µl Magnesiumchloridlösung (20 mM), 1 U einer thermostabilen DNA-Polymerase, bspw. Taq DNA-Polymerase, 1 µl einer bspw. 25 mM Lösung des PCR-Primers t3B (5'-AGGTCGCGGTTCGAATCC-3'; SEQ ID-Nr. 36). Der Primer ist so ausgestaltet, dass er an den Mikrosatelliten t3B binden kann. Der Ansatz wird mit Wasser auf 25 µl aufgefüllt.

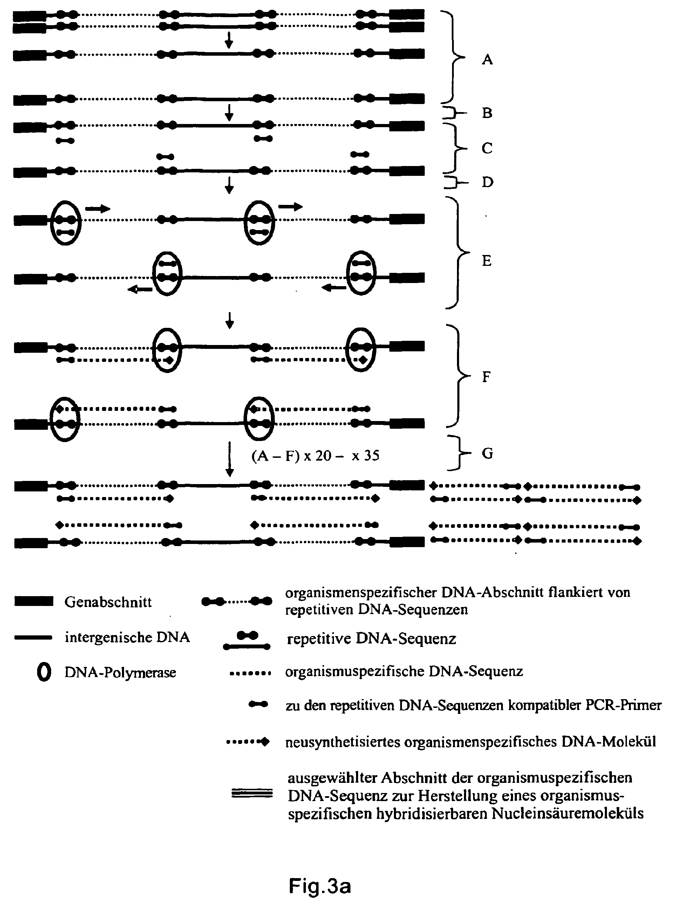

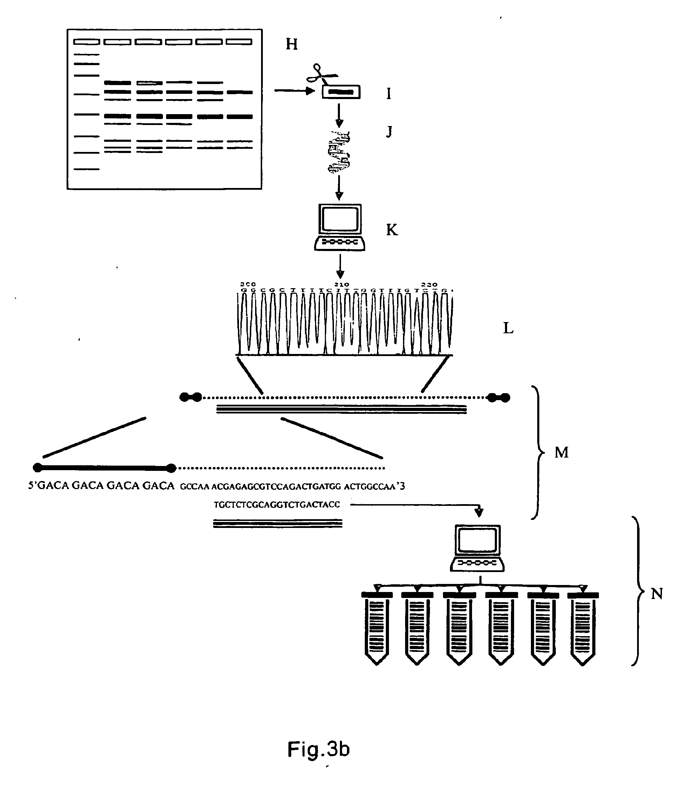

Es folgt die Durchführung der PCR. Die einzelnen Schritte sind schematisch in der Fig. 3 dargestellt.

Die Inkubation in einem Thermocycler erfolgt gemäß der gerätespezifischen Modalitäten. Zunächst erfolgt bspw. eine Inkubation, bspw. bei 94°C für 2 min. Anschließend erfolgt eine Inkubation bei bspw. 94°C, für 1 min. Dadurch wird der DNA-Doppelstrang aufgeschmolzen; vgl. Fig. 3A. Anschließend wird der Ansatz auf eine Temperatur abgekühlt, bspw. auf 56°C für 1 min (Fig. 3B), bei der die Primer an den Mikrosatelliten t3B hybridisieren können; vgl. Fig. 3C. Anschließend erfolgt eine Inkubation des Ansatzes bei der Arbeitstemperatur der Polymerase, bspw. bei 72°C für 2 min; vgl. Fig. 3D. Die Bindung der Polymerase ist in Teilabbildung Fig. 3E dargestellt. Es kommt zur Synthese von neuen für *Peronospora swinglei* s.l. spezifischen, d.h. organismenspezifischen DNA-Fragmenten, die von den repetitiven DNA-Sequenzen des t3B-Mikrosatelliten flankiert werden; vgl. Fig. 3F.

Die Schritte A bis F der Fig. 3 werden anschließend 20 bis 30 Mal wiederholt; vgl. Fig. 3G.

Die in der PCR erhaltenen für *Peronospora swinglei* s.l. spezifischen Amplifikate werden aufgetrennt, bspw. mittels Gelelektrophorese in einem Agarosegel; vgl. Fig. 3H. Dieses kann bspw. hergestellt werden, indem 1 g Agarose in ein Gefäß gegeben wird, auf 100 µl mit einer ionenhaltigen Flüssigkeit aufgefüllt wird, bspw. mit Tris-Borat-EDTA-Puffer (TBE). Danach wird die PCR-Reaktionslösung in eine präformierte Vertiefung des Agarosegels gegeben, bspw. nach Versetzen mit 1/6 vol. Glycerin und ggf. Farbstoffen, die bei der Elektrophorese in die gleiche Richtung laufen wie die DNA-Fragmente im Agarosegel und der Kontrolle des Fortschreitens der Elektrophorese diesen. Die Elektrophorese selbst erfolgt in einer dafür vorgesehenen Elektrophoresekammer, die mit einer zum Gel isotonischen Ionenhaltigen Lösung gefüllt ist, bevorzugterweise mit derselben die zur Herstellung des Agarosegels verwandt worden ist. Durch Anlegen einer Spannung, bspw. 140 V, wird die Wanderung der DNA-Fragmente durch das Agarosegel erzwungen, die durch das selektive Rückhaltevermögen, welches insbesondere auf der Größe der wandernden Fragmente beruht, aufgetrennt werden. Anschließend erfolgt eine Färbung mit einem an Nucleinsäuren assoziierenden oder bindenden Farbstoff, bspw. Ethidiumbromid, bspw. durch inkubieren des Agarosegels für eine halbe Stunde in einem ethidiumbromidhaltigen Färbebad.

### 4. Isolierung eines Amplifikates

Eine besonders gut amplifizierte Bande, hier bspw. im Bereich von 400 bis 900 bp, wird aus dem Agarosegel ausgeschnitten; vgl. Fig. 3I.

Die für *Peronospora swinglei* s.l. spezifische Nucleinsäure, die in dieser Bande enthalten ist, wird eluiert, bspw. mittels eines gängigen Gelextraktionskits, bspw. des QIAquick Gel Extraction Kits, nach Maßgabe des Herstellers QIAGEN; vgl. Fig. 3J.

Es folgt ein Einbringen und Vervielfältigen des erhaltenen Nucleinsäuremoleküls in einen PCR-Fragment-Klonierungvektor mittels eines handelüblichen PCR-Fragment-Klonierungskits, bspw. des TOPO TA Cloning Kits der Firma Invitrogen nach Maßgabe des Herstellers.

Der Vektor nebst darin eingeschlossenem für *Peronospora swinglei* s.l. spezifischem Nucleinsäurefragment werden isoliert, bspw. mittels eines handelsüblichen Plasmidpräparationskits, wie dem GeneElute HP Plasmid Midiprep Kits der Firma Sigma, entsprechend den Anweisungen des Herstellers.

### 5. Sequenzierung des isolierten Nucleinsäuremoleküls

Es folgt eine Sequenzierung des in den Vektor eingeschlossenen für *Peronospora swinglei* s.l. spezifischen Nucleinsäurefragmentes, und zwar mittels vektorspezifischer Sequenzierprimer und gängiger Verfahren, bspw. mittels des BigDye v3.1 Terminator Cycle Sequencing Kits von Applied Biosystems, gemäß den Angaben des Herstellers und anschließender Aufklärung der Sequenz des Nucleinsäuremoleküls, bspw. mittels eines Kapillarsequenziergerätes, bspw. des Geräts Abi-Prism 3730x1 DNA Analyzers von Applied Biosystems, gemäß der Angaben des Herstellers; vgl. Fig. 3K. Ein Ausschnitt des Chromatogramms der Sequenzierung ist in der Teilabbildung Fig. 3L dargestellt.

Im Falle dieses Ausführungsbeispiels wurde die für *Peronospora swinglei* s.l. spezifische Nucleotidsequenz SEQ ID-Nr. 2 ermittelt.

In der Tabelle 1 werden einige weitere Beispiele von Nucleotidsequenzen aus dem Genom von verschiedenen Pflanzenpathogenen gegeben, die sowohl 5'- als auch 3'-wärts von Mikrosatelliten wie dem t3B, oder Single Sequence Repeats (SSRs) flankiert werden.

**Tabelle 1: Beispiele von Nucleinsäuremolekülen bzw. Nucleotidsequenzen, die zur Herstellung eines organismusspezifischen hybridisierbaren Nucleinsäuremoleküls geeignet sind.**

| **Organismus** | **Flankierende Elemente** | **Hochvariables Fragment [SEQ ID-Nr.]** |
|---|---|---|
| *Peronospora arborescens* | Mikrosatellit t3B; beidseitig | 1 |
| *Peronospora swinglei* s.l. | Mikrosatellit t3B; beidseitig | 2 |
| *Fusarium oxysporum* | Single Sequence Repeats (SSRs); beidseitig, Sequenz: [GACA]₄ | 3 |
| *Fusarium oxysporum* | Mikrosatellit t3B; beidseitig | 4 |
| *Plasmopara petroselini* | Mikrosatellit t3B; beidseitig | 5 |
| *Plasmopara petroselini* | Single Sequence Repeats (SSRs); beidseitig, Sequenz: [GACA]₄ | 6 |
| *Peronospora valerianellae* | Single Sequence Repeats (SSRs); beidseitig, Sequenz: [GACA]₄ | 7 |
| *Peronospora valerianellae* | Single Sequence Repeats (SSRs); beidseitig, Sequenz: [GACA]₄ | 8 |

### 6. Auswahl eines zur Herstellung eines spezifischen hybridisierbaren Nucleinsäuremoleküls geeigneten Abschnitts

Aus der in Schritt 5 aufgeklärten Nucleotidsequenz des für *Peronospora swinglei* s.l. spezifischen Nucleinsäuremoleküls wurden zwei Abschnitte ausgewählt, die zur Herstellung von PCR-Primern geeignet sind; vgl. Fig. 3M. Die in Schritt 5 aufgeklärte Nucleotidsequenz dient demnach als Matrize zur Herstellung von zwei hybridisierbaren Nucleinsäuremolekülen, welche als PCR-Primer zum Nachweis einer Infektion einer Pflanze mit *Peronospora swinglei* s.l. eingesetzt werden können.

Die Nucleotidfolgen werden so gewählt, dass sie nach Stand der Wissenschaft spezifisch hybridisieren und auch nach Datenbankabgleich (GenBank) organismus-d.h. *Peronospora swinglei* s.l.-spezifisch sind, da sie sich einzeln in Teilen nur in wenigen und kombiniert in keiner Sequenz eines in GenBank katalogisierten Nucleinsäuremoleküls wiederfinden.

### 7. Herstellung eines oder mehrerer Nucleinsäuremoleküle, das den in Schritt (6) ausgewählten Abschnitt aufweist

Die Herstellung der zwei PCR-Primer, die zum Nachweis einer Infektion einer Pflanze mit *Peronospora swinglei* s.l. eingesetzt werden können, erfolgt bspw. mittels einer 96 Column DNA Synthesizer Workstation der Firma PolyGen nach Maßgabe des Herstellers; vgl. Fig. 3N. Zahlreiche kommerzielle Anbieter, bspw. die Firma Sigma-Aldrich, bieten die Herstellung von Nucleotidoligomeren und - polymeren an.

Das hybridisierbare Nucleinsäuremolekül, das als S_Ps_F bezeichnet wurde und als Vorwärts-PCR-Primer eingesetzt werden kann, weist die Sequenz 5'-TGCTCTCGCAGGTCTGACTACC-3'auf (SEQ ID-Nr. 11). Das hybridisierbare Nucleinsäuremolekül, das als S_Ps_R bezeichnet wurde und als Rückwärts-PCR-Primer eingesetzt werden kann, weist die Sequenz 5'-cacgcaaatccctacctctgcc-3'auf (SEQ ID-Nr. 12).

Zwei weitere hybridisierbare Nucleinsäuremoleküle wurden als Primer für eine verschachtelte oder nested-PCR ausgestaltet, bei der eine zweite Amplifizierung basierend auf der ersten Amplifizierung genutzt wird, um die Empfindlichkeit noch weiter zu erhöhen. Das hybridisierbare Nucleinsäuremolekül, das als S_Ps_Fn bezeichnet wurde und als Vorwärts-PCR-Primer eingesetzt werden kann, weist die Sequenz 5'-GTCTTAACGAGACCGCGTGGTC-3' (SEQ ID-Nr. 13) auf. Das hybridisierbare Nucleinsäuremolekül, das als S_Ps_Rn bezeichnet wurde und als Rückwärts-PCR-Primer eingesetzt werden kann, weist die Sequenz 5'-ATGACGGCAGGTGCTGTCGTTC-3' (SEQ ID-Nr. 14) auf.

In der nachstehenden Tabelle 2 sind einige Beispiele von PCR-Primern gelistet, die mittels des erfindungsgemäßen Verfahrens generiert wurden.

**Tabelle 2: Beispiele von speziespezifischen hybridisierbaren Nucleinsäuremolekülen zum Nachweis von ausgewählten Pflanzenpathogenen.**

| **Organismusspezifität [Organismus]** | **PCR-Primer [Sequenz]** | **SEQ ID-Nr.** |
|---|---|---|
| *Peronospora arborescens* | Vorwärtsprimer (S_Pa_F): | |
| | 5'-ACGCCCTTTCGGACCAG-3' | 9 |
| | Rückwärtsprimer (S_Pa_R): | |
| | 5'-CAGCATGCGCCAGAAGAT-3' | 10 |
| *Peronospora swinglei* s.l., | Vorwärtsprimer (S_Ps_F): | |
| Nachweis 1 | 5'-TGCTCTCGCAGGTCTGACTACC -3' | 11 |
| | Rückwärtsprimer (S_Ps_R): | |
| | 5'-CACGCAAATCCCTACCTCTGCC-3' | 12 |
| *Peronospora swinglei* s.l. | Vorwärtsprimer (S_Ps_Fn): | |
| Nachweis 2 (nested-PCR in Verbindung mit Nachweis 1) | 5'-GTCTTAACGAGACCGCGTGGTC-3' | 13 |
| | Rückwärtsprimer(S_Ps_Rn): | |
| | 5'-ATGACGGCAGGTGCTGTCGTTC-3' | 14 |
| *Plasmopara halstedii* | Vorwärtsprimer (S_P1-halst_F): | |
| | 5'-CTC TGT CCA CGC ATT CCG-3' | 15 |
| | Rückwärtsprimer (S_P1-halst_R): | |
| | 5'-GTT TGT ATC TGC TTA AAC TGA C-3' | 16 |
| *Fusarium oxysporum forma specialis strigae* | Vorwärtsprimer1 (Detect_Foxy-Psm_F1): | |
| | 5'-GGG TTC CCA ATC TCA ATC-3' | 17 |
| | Vorwärtsprimer2: | |
| | 5'-ACT CAC TAC GCC TCG GTC-3' | 18 |
| | Rückwärtsprimer (Detect_Foxy-Psm_R): | |
| | 5'-TCG ACA GTC TTT AAG TCT TG-3' | 19 |
| *Plasmopara petroselini* Nachweis 1 | Vorwärtsprimer (S_P1_petro_F1) | |
| | 5'-ACCAAGAGCGCCTAACTTGACGCT-3' | 20 |
| | Rückwärtsprimer (S_P1_petro_R1) | 21 |
| | 5'-GGGTTCGAATCCGGGGCATCTCGT-3' | |
| *Plasmoparapetroselini* Nachweis 2 (nested-PCR in Verbindung mit Nachweis 1) | Vorwärtsprimer (S_P1_petro_F1n) | 22 |
| | 5'-CCGTCAACTCATAGGCGAATGCA-3' | |
| | Rückwärtsprimer (S_P1_petro_R1n) | 23 |
| | 5'-CACTAAACCAACGGCGCTTGCACG-3' | |
| *Plasmopara petroselini* | Vorwärtsprimer (S_P1_petro_F2) | 24 |
| Nachweis 3 | 5'-CGCATTATCGCTCTAGGAAGCGAC-3' | 25 |
| | Rückwärtsprimer (S_P1_petro_R2) | |
| | 5 '-GGCCCGAATGTGATGTGCCGCGTT-3' | |
| *Plasmopara petroselini* | Vorwärtsprimer (S_P1_petro_F2n) | 26 |
| Nachweis 4 (nested-PCR in Verbindung mit Nachweis 3) | 5'-CCAGCTACGCACATGGAACGACCT-3' | 27 |
| | Rückwärtsprimer (S_P1_petro_R2n) | |
| | 5'-ACGCAAGAATCATACCTCGCTGC-3' | |
| *Peronospora valerianellae* | Vorwärtsprimer (S_Per_val_F1) | |
| Nachweis 1 | 5'-GACAGATTGCGTTACCCACTGGCT-3' | 28 |
| | Rückwärtsprimer (S_Per_val_R1) | 29 |
| | 5'-CCGACAAAACGTGTTGCTGCTGCA-3' | |
| *Peronospora valerianellae* | Vorwärtsprimer (S_Per_val_F1n) | 30 |
| | 5'-TTGCGTCGACGTTGGCGATGCACG-3' | |
| Nachweis 2 (nested-PCR in Verbindung mit Nachweis 1) | Rückwärtsprimer (S_Per_val_R1n) | 31 |
| | 5'-GAGCGCTCTAAGTGAGTGTCTCGT-3' | 32 |
| | Vorwärtsprimer (S_Per_val_F2) | |
| *Peronospora valerianel-lae* | 5'-GACGTACAAATCACCCCGATGTCG-3' | |
| Nachweis 3 | Rückwärtsprimer (S_Per_val_R2) | |
| | 5'-TGCTGACGTTCACAAGCGCTA-3' | 34 |
| | Vorwärtsprimer (S_Per_val_F2n) | |
| *Peronospora valerianellae* | 5'-GATACTCCGGCATGACAGTGTCGT-3' | 35 |
| | Rückwärtsprimer (S_Per_val_R2n) | |
| Nachweis 4 (nested-PCR in Verbindung mit Nachweis 3) | 5'-GGGATTTGCGGTAACCCTGTCAGC-3' | |

### Beispiel 2: Verwendung der erfindungsgemäßen Nucleinsäuremoleküls (PCR-Primer) zum spezifischen Nachweis von Peronospora swinglei s.l. in Basilikumsaatgut

Das Nachweisverfahren umfasst im Wesentlichen folgende 5 Arbeitsschritte: (a) Aufschluss des Saatgutes; (b) DNA-Extraktion; (c) Qualitätsprüfung der gewonnenen DNA-Lösung; (d) Anwendung des PCR-basierten Nachweises und (e) elektrophoretische Trennung der PCR-Reaktionslösungen.

### (a) Aufschluss des Saatgutes

Um eine innere und äußere Kontamination mit dem Erreger erfassen zu können, wurde das Basilikumsaatgut von sieben unterschiedlichen Saatgutpartien vor der Extraktion für 5 min unter flüssigem Stickstoff mechanisch aufgeschlossen. Alternativ sind auch andere Aufschlusstechniken einsetzbar, wie z.B. das Zerkleinern in Mühlen, Mixern oder der enzymatische Abbau der strukturgebenden Zellbestandteile.

Anschließend wurden 5 g (~ ∅ 1000 Korngewicht von Basilikumsamen) des zerkleinerten Saatgutes in ein steriles 50 ml Zentrifugationsröhrchen überführt.

### (b) DNA-Extraktion (nach Möller et al. 1992, modifiziert)

Nach der gründlichen Suspendierung in 20 ml SSB Puffer (Tris: 200 mM, NaCl: 300 mM, EDTA: 25 mM, SDS: 1 % (v/w)) wurde das Gemisch unter leichtem Schütteln für 30 min bei Raumtemperatur inkubiert. Nach der Sedimentation der unlöslichen Bestandteile durch eine fünfminütige Zentrifugation bei mindestens 9.000 s/t²*9,81 wurden 5 ml der überstehenden Lösung abgehoben und in ein neues 50 ml Zentrifugationsröhrchen überführt. Die restlichen 15 ml des SSB Puffers wurden nahezu vollständig von den stark quellenden Inhaltsstoffen der Basilikumsamen aufgenommen. Nach dem Hinzufügen von 625 µl einer 4 M Kaliumacetatlösung wurde die Extraktionslösung für 30 min unter leichtem Schütteln auf Eis inkubiert. Nach der Sedimentation der unlöslichen Bestandteile durch eine fünfminütige Zentrifugation bei mindestens 9.000 s/t²*9,81 wurde der flüssige Überstand vollständig in ein neues Zentrifugationsröhrchen überführt. Nach dem Hinzufügen von 0,6 Volumenanteilen Isopropanol (100%) wurde die Extraktionslösung für 10 min unter leichtem Schütteln bei Raumtemperatur inkubiert. Durch eine 10-minütige Zentrifugation bei mindestens 9.000 s/t²*9,81 wurde die nun unlösliche DNA sedimentiert. Nach dem vollständigen Abheben des Überstandes wurde das Sediment zweimal mit Ethanol (70%) gewaschen und anschließend getrocknet.

Das getrocknete Sediment wurde in 5 ml TE-Puffer resuspendiert und anschließend mit 500 µl einer 1%-igen Spermidin-Lösung versetzt. Nach einer 15-minütigen Inkubation auf Eis wurden die unlöslichen Bestandteile durch 10-minütige Zentrifugation sedimentiert, der Überstand wurde verworfen. Nach der Resuspension des Sedimentes durch Zugabe von 1 ml Spermidine Removal Buffer (Natriumacetat: 300 mM; Magnesiumacetat: 10 mM) und 3 ml kaltem Ethanol (100%) folgt eine Inkubationsphase von 1 Stunde auf Eis, unter gleichzeitigem leichten Schütteln. Durch eine 10-minütige Zentrifugation bei mindestens 9.000 s/t²*9,81 wurde die unlösliche DNA sedimentiert. Nach dem vollständigen Abheben des Überstandes wurde das Sediment zweimal mit Ethanol (70%) gewaschen und anschließend getrocknet.

Zum Schluss wurde die isolierte und gereinigte DNA in 500 µl H₂O aufgenommen, in ein 1,5 ml Reaktionsgefäß überführt und bis zur weiteren Verwendung bei -18°C aufbewahrt.

### (c) Qualitätsprüfung der gewonnenen DNA-Lösung

### Messungen:

Die Konzentration und die Reinheit der isolierten DNA-Moleküle wurden mit Hilfe der Absorptionsmessung bestimmt. Die Konzentrationsbestimmung resultiert aus dem gemessenen Wert der optischen Absorption im UV-Bereich bei 260 nm. Die Reinheit der isolierten DNA wurde aus dem Verhältnis der Absorptionsmessung bei 260 und 280 nm bestimmt (BioPhotometer, Eppendorf, Germany). Parallel wurde die Qualität der isolierten DNA durch elektrophoretische Auftrennung im Agarosegel (1 %) ermittelt.

Im Falle von gravierenden Verunreinigungen wurde die DNA mit Hilfe eines handelsüblichen DNA-Reinigungs-Kits, wie es z.B. von GE Healthcare in Form des "illustra GFX™ PCR DNA and Gel Band Purification Kit" angeboten wird, nachgereinigt und nochmals geprüft.

### Kontroll-PCR:

Zur Kontrolle der Amplifizierbarkeit der gewonnen DNA wurde eine PCR mit Primern für einen Teilbereich der Chloroplasten-DNA von Basilikum durchgeführt. Nur DNA-Lösungen, die im Agarosegel der Kontroll-PCR ein eindeutiges Amplifikat in der Größe von 520 nt zeigen, werden für den PCR-gestützten Nachweis des Erregers zugelassen. Durch dieses Vorgehen können falsch-negative Ergebnisse ausgeschlossen werden.

### (d) Anwendung des PCR-basierten Nachweises

### Nachweisreaktion 1

Eine PCR-Reaktion wird in einem PCR-geeigneten Reaktionsgefäß angesetzt, bspw. bestehend aus 2,5 µl 10x PCR-Puffer, 2,5 µl einer 2 mM Lösung der vier Desoxynucleotidtriphosphate (dNTPs), 2 µl Magnesiumchloridlösung (20 mM), 1 U einer thermostabilen DNA-Polymerase, bspw. Taq DNA-Polymerase, 1 µl einer bspw. 25 mM Lösung des PCR-Primers S_Ps_F (5'-TGCTCTCGCAGGTCTGACTACC -3'; SEQ ID-Nr. 11) und S Ps R (5'-CACGCAAATCCCTACCTCTGCC-3'; SEQ ID-Nr. 12). Die Primer sind so ausgestaltet, dass sie an den identifizierten, für *Peronospora swinglei* s.l. spezifischen, intergenischen DNA-Abschnitt (SEQ ID-Nr. 2) binden können. Der Ansatz wird mit Wasser auf 25 µl aufgefüllt.

Es erfolgt die Durchführung der PCR, bspw. mit folgenden Bedingungen: Die Inkubation in einem Thermocycler erfolgt gemäß den gerätespezifischen Modalitäten. Zunächst erfolgt bspw. eine Inkubation bei 94°C für 5 min. Anschließend erfolgt eine Inkubation bei bspw. 94°C für 30 sec. Danach eine Inkubation bei 65°C für 30 sec. Anschließend erfolgt eine Inkubation bei 72°C für 40 sec. Die letzten drei Schritte werden anschließend bspw. 45 Mal wiederholt. Es kann dann eine abschließende Inkubation bei 72°C für 4 min durchgeführt werden.

### Nachweisreaktion 2

Es folgt eine zweite Nachweisreaktion (nested-PCR). Hierzu werden 1 µl der ersten Nachweisreaktion und 1 µl einer bspw. 25 mM Lösung der nested Primer S_Ps_Fn (5'-GTCTTAACGAGACCGCGTGGTC-3' SEQ ID-Nr. 13) und S_Ps_Rn (5'-ATGACGGCAGGTGCTGTCGTTC-3' SEQ ID-Nr. 14) zu den übrigen in Nachweis 1 beschrieben Lösungen bzw. Reagenzien (10x PCR-Puffer, dNTPs, Magnesiumchloridlösung und Taq DNA-Polymerase) in einem neuen PCR-geeigneten Reaktionsgefäß gemischt. Die Inkubation in einem Thermocycler erfolgt gemäß den gerätespezifischen Modalitäten. Zunächst erfolgt bspw. eine Inkubation bei 94°C für 5 min. Anschließend erfolgt eine Inkubation bei bspw. 94°C, für 30 sec. Danach eine Inkubation bei 65°C für 30 sec. Anschließend erfolgt eine Inkubation bei 72°C für 30 sec. Die letzten drei Schritte werden anschließend bspw. 35 Mal wiederholt. Es kann dann eine abschließende Inkubation bei 72°C für 4 min durchgeführt werden.

### (e) Elektrophoretische Trennung der PCR-Reaktionslösungen

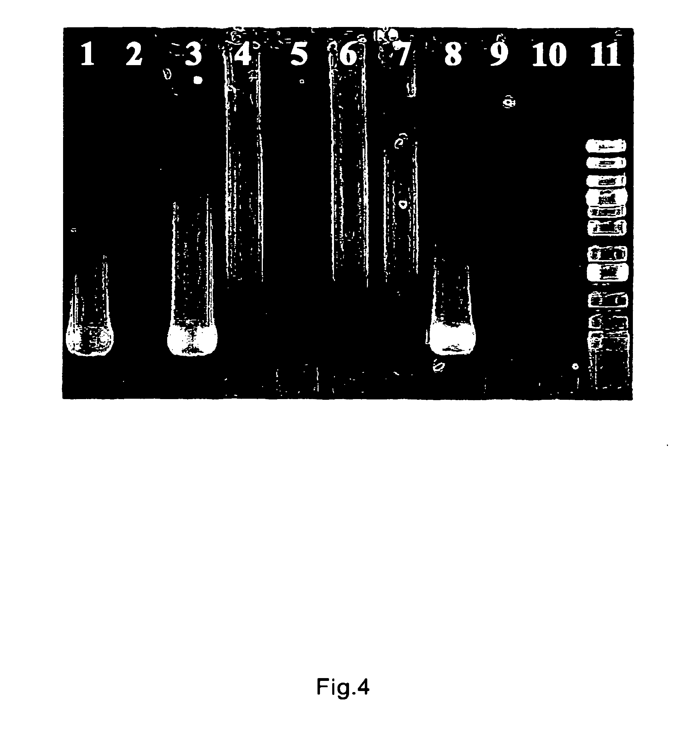

Die in der Nachweisreaktion 2 erhaltenen Amplifikate werden aufgetrennt, bspw. mittels Gelelektrophorese, bspw. in einem Agarosegel. Dieses kann bspw. hergestellt werden, indem 1 g Agarose in ein Gefäß gegeben werden, auf 100 µl mit einer ionenhaltigen Flüssigkeit aufgefüllt wird, bspw. mit Tris- Borat-EDTA-Puffer (TBE). Danach wird die PCR-Reaktionslösung in eine präformierte Vertiefung des Agarosegels gegeben, bspw. nach Versetzen mit 1/6 vol. Glycerin und ggf. Farbstoffen, die bei der Elektrophorese in die gleiche Richtung laufen wie die DNA-Fragmente im Agarosegel und der Kontrolle des Fortschreitens der Elektrophorese dienen. Die Elektrophorese selbst erfolgt in einer dafür vorgesehenen Elektrophoresekammer, die mit einer zum Gel isotonischen ionenhaltigen Lösung gefüllt ist, bevorzugterweise mit derselben, die zur Herstellung des Agarosegels verwandt worden ist. Durch Anlegen einer Spannung, bspw. 140 V, wird die Wanderung der DNA-Fragmente durch das Agarosegel erzwungen, die durch das selektive Rückhaltevermögen, welches insbesondere auf der Größe der wandernden Fragmente beruht, aufgetrennt werden. Anschließend erfolgt eine Färbung mit einem an Nucleinsäuren assoziierenden oder bindenden Farbstoff, bspw. Ethidiumbromid, bspw. durch inkubieren des Agarosegels für eine halbe Stunde in einem ethidiumbromidhaltigen Färbebad. Das Ergebnis ist in Fig. 4 dargestellt.

Die Spuren des 1,5 %igen Agarosegeles waren folgende: Spur 1: Saatgutprobe 1 (kontaminiert); Spur 2: Saatgutprobe 2; Spur 3: Saatgutprobe 3 (kontaminiert); Spur 4: Saatgutprobe 4; Spur 5: Saatgutprobe 5; Spur 6: Saatgutprobe 6; Spur 7: Saatgutprobe 7; Spur 8: Referenz DNA von *Peronospora swinglei* s.l (Positivkontrolle); Spur 9: Referenz DNA von *Ocimum basilicum* (Basilikum) (Negativkontrolle 1); Spur 10: PCR-Ansatz ohne DNA (Negativkontrolle 2); Spur 11: DNA-Größenmarker (Standard)1.

Die Referenzprobe von *Peronospora swinglei* s.l. (Spur 8) zeigt ein deutlich sichtbares Amplifikat in der Größe von 500 nt (A). Die weiße Bande ist das organismusspezifische hybridisierbare Nucleinsäuremolekül von *Peronospora swinglei* s.l.. Das gleiche Amplifikat findet sich in den PCR-Ansätzen der Saatgutproben 1 (Spur 1) und 3 (Spur 3), nicht vorhanden ist das Amplifikat in den Saatgutproben 2, 4 - 7 (Spuren 2, 4 -7) und in den Negativkontrollen 1 und 2 (Spuren 9 und 10).

Mit dem erfindungsgemäßen Verfahren konnte somit der Erreger *Peronospora swinglei* s.l. in den Saatgutproben 1 und 3 eindeutig nachgewiesen werden. Der in den Spuren 4, 6 und 7 auftretende Schmier ist eine, für nested-PCR Ansätze typische, unspezifische Nebenreaktion, die nicht mit dem spezifischen Amplifikat in den Spuren 1, 3 und 8 zu verwechseln ist. Falsch negative Reaktionen können durch die vorangegangene Qualitätsprüfung ausgeschlossen werden. Falsch positive Reaktionen werden durch die Mitführung der Negativkontrollen 1 und 2 ausgeschlossen.

Wie im Praxisbeispiel gezeigt, führt die Anwendung des erfindungsgemäßen Verfahrens in Form eines PCR-gestützten Nachweises zu einem eindeutig interpretierbaren Ergebnis. Die mit dem Erreger *Peronospora swinglei* s.l. kontaminierten Saatgutpartien können sicher identifiziert und vom Markt genommen werden. Somit ist die Verbreitung des Zielorganismus, in diesem Falle eines gefährlichen Schaderregers unterbunden.

**Tabelle 3: Vergleich eines klassischen DNA-Fingerprintings mit dem erfindungsgemäßen Nachweisverfahren**

| | | Musteranalyse | Erfindungsgemäßes Verfahren |
|---|---|---|---|
| | Nachweisprinzip | Klassisches DNA-Fingerprinting | Organismusspezifische hybridisierbare Nucleinsäuremoleküle (z.B. PCR-Primer) |
| 1 | Organismus muss in Reinkultur vorliegen | Ja | Nein |
| 2 | Bearbeitungszeit | 3 Tage bis mehrere Wochen | 2 Tage |
| 3 | Interpretation der Test-ergebnisse | schwierig und zeitaufwändig | Einfach (Ja/Nein Antwort) |
| 4 | Vergleichbarkeit der Testergebnisse | Nur sehr bedingt möglich | Ja |
| 5 | Handhabung | Aufwändig | Einfach |
| 6 | Einarbeitungsaufwand | Groß | Gering |
| 7 | In Kit umsetzbar | Nur sehr bedingt | Ja |
| 8 | Entwicklungszeit | Sehr lang | kurz |
| 9 | genomweites Screening | Ja | Ja |
| 10 | Differenzierung zwischen nahe verwandten Spezies | möglich | möglich |
| 11 | Als Sonden einsetzbar | Nein | Ja |
| 12 | Identifikation neuer differenzierender DNA-Abschnitte | extrem aufwändig | Einfach und schnell möglich |

Wie die Tabelle 3 zeigt, weist das erfindungsgemäße Nachweisverfahren gegenüber der Musteranalyse eine Vielzahl von Vorteilen auf. Es ist insbesondere gekennzeichnet durch seine flexible Nutzung (Zeile 11) und seine hohe Anpassungsfähigkeit an neue Organismen (Zeile 12). Durch das erfindungsgemäße Herstellungsverfahren ist der Anwender in der Lage, innerhalb von kurzer Zeit neue organismusspezifische Nachweisverfahren zu entwickeln und diese praxistauglich zur sicheren Identifikation von Organismen einzusetzen.

## Patentansprüche

1. Verfahren zur Auswahl eines für pflanzenpathogene Organismen spezifischen hybridisierbaren Nucleinsäuremoleküls, das folgende Schritte aufweist:
1. Bereitstellung einer aus einem pflanzenpathogenen Organismus stammenden genomischen doppelsträngigen DNA;
2. Erstellung eines genetischen Fingerabdrucks von der genomischen doppelsträngigen DNA zum Erhalt eines Musters von Amplifikaten von Genomabschnitten durch folgende Schritte:
2.1 Amplifizierung von Abschnitten der genomischen doppelsträngigen DNA des Organismus mittels Polymerase-Kettenreaktion (PCR) zum Erhalt einer Mischung von Amplifikaten, und
2.2 Vereinzelung der in *der* Mischung enthaltenen Amplifikate zum Erhalt eines Musters von Amplifikaten von Genomabschnitten;
3. Isolierung von zumindest einem Amplifikat aus dem Muster;
4. Sequenzierung des isolierten Amplifikats zum Erhalt einer Nucleotidsequenz;
5. Auswahl eines Abschnitts der Nucleotidsequenz von 3 bis 1000 Nucleotiden nach Datenbankabgleich, der zur Herstellung eines organismusspezifischen hybridisierbaren Nucleinsäuremoleküls geeignet ist,
wobei im Rahmen der PCR als Vorwärts-Primer ein solcher gewählt wird, der an eine Nucleotidsequenz einer Satelliten-DNA auf dem ersten DNA-Strang des Organismus hybridisiert, und als Rückwärts-Primer ein solcher gewählt wird, der an eine Nucleotidsequenz einer Satelliten-DNA auf dem zu dem ersten DNA-Strang komplementären zweiten DNA-Strang des Organismus hybridisiert, und
wobei die Nucleotidsequenz der Satelliten-DNA auf dem ersten DNA-Strang identisch ist zu der Nueleotidsequenz der Satelliten-DNA auf dem zweiten DNA-Strang, und
wobei im Schritt (3) aus dem Muster von Amplifikaten von Fragmenten der genomischen Information ein solches Amplifikat isoliert wird, das in Relation zu den übrigen Amplifikaten bei der Amplifizierung überdurchschnittlich stark amplifiziert wurde.

2. Verfahren nach Anspruch 1, **dadurch gekennzeichnet, dass** die Satelliten-DNA ausgewählt ist aus der Gruppe bestehend aus: Mikrosatelliten-DNA, simple sequence repeats (SSRs) und intersimple sequence repeats (ISSRs).

3. Verfahren nach Anspruch 1 oder 2, **dadurch gekennzeichnet, dass** die Vereinzelung der in der Mischung enthaltenen Amplifikate mittels einer chromatographischen und/oder elektrophoretischen Methode erfolgt.

4. Verfahren nach einem der Ansprüche 1 bis 3, **dadurch gekennzeichnet, dass** nach der Isolierung von zumindest einem Amplifikat aus dem Muster und vor der Sequenzierung des isolierten Amplifikats folgende weitere Schritte erfolgen:
a) Einfügung des isolierten Amplifikats in einen Vektor,
b) Einbringen des Vektors in einen geeigneten Organismus, und
c) Isolieren des Vektors aus dem Organismus zum Erhalt von zumindest einem isolierten Amplifikat.

5. Verfahren nach einem der Ansprüche 1 bis 4, **dadurch gekennzeichnet, dass** der zur Herstellung eines organismusspezifischen hybridisierbaren Nucleinsäuremoleküls geeignete ausgewählte Abschnitt eine Länge von 10 bis 64, vorzugsweise 12 bis 48, weiter vorzugsweise 14 bis 36, weiter vorzugsweise 16 bis 27 Nucleotiden aufweist.

6. Verfahren nach einem der Ansprüche 1 bis 5, **dadurch gekennzeichnet, dass** das hergestellte Nucleinsäuremolekül als PCR-Primer ausgestaltet wird.

7. Verwendung einer intergenischen Nucleotidsequenz, die zwischen zwei Elementen der genetischen Information eines Organismus liegt und die eine der Nucleotidsequenzen SEQ ID Nr. 9 bis 14 und 17 bis 35 aufweist, zur Herstellung eines für pflanzenpathogene Organismen spezifischen hybridisierbaren Nucleinsäuremoleküls, **dadurch gekennzeichnet, dass** die komplementären DNA-Stränge der zwei Elemente jeweils eine identische Satelliten-DNA-Nucleotidsequenz aufweisen.

8. Verwendung nach Anspruch 7, **dadurch gekennzeichnet, dass** das für pflanzenpathogene Organismen spezifische hybridisierbare Nucleinsäuremolekül ein PCR-Primer ist.

9. Nucleinsäuremolekül, das aus einer der Nucleotidsequenzen SEQ ID Nr. 9 bis 14 und 17 bis 35 aus dem beiliegenden Sequenzprotokoll oder einer hierzu komplementären Nucleotidsequenz besteht.

## Claims

1. Method for selecting a hybridizable nucleic acid molecule which is specific for plant pathogenic organisms, comprising the following steps:
1. Providing a genomic double-stranded DNA originating from a plant pathogenic organism;
2. establishing a genetic fingerprint from the genomic double-stranded DNA to obtain a pattern of amplificates of genome segments by the following steps:
2.1 amplifying of segments of the genomic double-stranded DNA of the organism by means of a polymerase chain reaction (PCR) to obtain a mixture of amplificates, and
2.2 separating the amplificates contained in the mixture to obtain a pattern of amplificates of genome segments;
3. isolating of at least one amplificate from the pattern;
4. sequencing of the isolated amplificate to obtain a nucleotide sequence;
5. selecting of a segment of the nucleotide sequence of 3 to 1000 nucleotides after data base comparison, which is suitable for the production of an organism-specific hybridizable nucleic acid molecule,
wherein in the context of the PCR such a forward primer is selected, which hybridizes to a nucleotide sequence of a satellite DNA on the first DNA strand of the organism, and such a backward primer is selected, which hybridizes to a nucleotide sequence of a satellite DNA on the second DNA strand of the organism, which is complementary to the first DNA strand, and
wherein the nucleotide sequence of the satellite DNA on the first DNA strand is identical with the nucleotide sequence of the satellite DNA on the second DNA strand, and
wherein in step (3) such an amplificate is isolated from the pattern of amplificates which, in relation to the remaining amplificates, was amplified during the amplification in an above-average amount.

2. Method according to claim 4, **characterized in that** the satellite DNA is selected from the group consisting of: microsatellite DNA, simple sequence repeats (SSRs) and intersimple sequence repeats (ISSRs).

3. Method according to claims 1 or 2, **characterized in that** the separating of the amplificates contained in the mixture is performed by means of a chromatographic and/or electrophoretic method.

4. Method according to any of claims 1 to 3, **characterized in that** after the isolation of at least one amplificate from the pattern and before the sequencing of the isolated amplificate the following further steps are performed:
a) insertion of the isolated amplificate into a vector,
b) introduction of the vector into an appropriate organism, and
c) isolation of the vector from the organism to obtain at least one isolated amplificate.

5. Method according to any of claims 1 to 4, **characterized in that** the selected segment suitable for the production of an organism-specific hybridizable nucleic acid molecule comprises a length of 10 to 64, preferably 12 to 48, further preferred 14 to 36, further preferred 16 to 27 nucleotides.

6. Method according to any of the claims 1 to 5, **characterized in that** the produced nucleic acid molecule is designed as a PCR primer.

7. Use of an intergemetic nucleotide sequence which is located between two elements of the genetic information of an organism, and which comprises any of the nucleotide sequences of SEQ ID nos. 1 to 14 and 17 to 35, for the production of a hybridizable nucleic acid molecule which is specific for plant pathogens, **characterized in that** each of the complementary DNA strands of the two elements comprises the identical satellite DNA nucleotide sequence.

8. Use according to claim 7, **characterized in that** the organism-specific hybridizable nucleic acid molecule is a PCR primer.

9. Nucleic acid molecule consisting of any of the nucleotide sequences of SEQ ID no. 9 to 14 from the enclosed sequence listing, or a nucleotide sequence complementary thereto.

## Revendications

1. Procédé de sélection d'une molécule d'acide nucléique hybridable spécifique d'organismes pathogènes de plantes, qui présente les étapes suivantes :
1. mise à disposition d'un ADN génomique à brin double provenant d'un organisme pathogène de plantes ;
2. production d'une empreinte génétique de l'ADN génomique à brin double pour obtenir un modèle d'amplicons des segments génomiques par les étapes suivantes :
2.1 amplification de segments d'ADN génomique à brin double de l'organisme au moyen d'une réaction en chaîne polymérase (PCR) pour obtenir un mélange d'amplicons, et
2.2 mise à l'écart des amplicons contenus dans le mélange pour obtenir un modèle d'amplicons des segments génomiques ;
3. isolement d'au moins un amplicon du modèle ;
4. séquençage de l'amplicon isolé pour obtenir une séquence nucléotidique ;
5. sélection d'un segment de la séquence nucléotidique de 3 à 1000 nucléotides après un rapprochement de bases de données qui est approprié pour la production d'une molécule d'acide nucléique hybridable spécifique d'un organisme,
dans lequel, dans le cadre de la PCR, on choisit comme amorce sens, une amorce qui s'hybride à une séquence nucléotidique d'un ADN satellite sur le premier brin d'ADN de l'organisme, et comme amorce anti-sens, une amorce qui s'hybride à une séquence nucléotidique d'un ADN satellite sur le deuxième brin d'ADN de l'organisme, complémentaire au premier brin d'ADN, et
dans lequel la séquence nucléotidique de l'ADN satellite sur le premier brin d'ADN est identique à la séquence nucléotidique de l'ADN satellite sur le deuxième brin d'ADN, et
dans lequel à l'étape (3), on isole du modèle d'amplicons de fragments de l'information génomique, un amplicon qui a été fortement amplifié, à un niveau supérieur à la moyenne, par rapport aux autres amplicons lors de l'amplification.

2. Procédé selon la revendication 1, **caractérisé en ce que** l'ADN satellite est choisi dans le groupe constitué d'ADN microsatellite, de répétitions de séquences simples (SSR) et de répétitions de séquences inter-simples (ISSR).

3. Procédé selon la revendication 1 ou 2, **caractérisé en ce que** la mise à l'écart des amplicons contenus dans le mélange s'effectue au moyen d'un procédé chromatographique et/ou électrophorétique.

4. Procédé selon l'une des revendications 1 à 3, **caractérisé en ce qu'**après l'isolement d'au moins un amplicon du modèle et avant le séquençage de l'amplicon isolé, les étapes suivantes sont réalisées :
a) insertion de l'amplicon isolé dans un vecteur,
b) intégration du vecteur dans un organisme approprié, et
c) isolement du vecteur de l'organisme pour obtenir au moins un amplicon isolé.

5. Procédé selon l'une des revendications 1 à 4, **caractérisé en ce que** le segment approprié sélectionné pour la production d'une molécule d'acide nucléique hybridable spécifique d'un organisme présente une longueur de 10 à 64, de préférence, de 12 à 48, de manière davantage préférée, de 14 à 36, de manière encore davantage préférée, de 16 à 27 nucléotides.

6. Procédé selon l'une des revendications 1 à 5, **caractérisé en ce que** la molécule d'acide nucléique produite est conçue comme une amorce de PCR.

7. Utilisation d'une séquence nucléotidique intergénique qui se trouve entre deux éléments de l'information génétique d'un organisme et qui présente l'une des séquences nucléotidiques SEQ ID N° : 9 à 14 et 17 à 35, pour la production d'une molécule d'acide nucléique hybridable spécifique d'organismes pathogènes de plantes, **caractérisée en ce que** les brins d'ADN complémentaires des deux éléments présentent respectivement une séquence nucléotidique d'ADN satellite identique.

8. Utilisation selon la revendication 7, **caractérisée en ce que** la molécule d'acide nucléique hybridable spécifique d'organismes pathogènes de plantes est une amorce de PCR.

9. Molécule d'acide nucléique qui est constituée de l'une des séquences nucléotidiques SEQ ID N° : 9 à 14 et 17 à 35 du protocole de séquence joint ou d'une séquence nucléotidique complémentaire de celle-ci.
